# EUROPEAN PATENT APPLICATION

(11) **EP 2 287 304 A1**
(43) Date of publication of application: **23.02.2011**
(21) Application number: 09010572.7
(22) Date of filing: 17.08.2009
(51) Int. Cl.: C12N 15/113, A61K 31/713

(54) **Novel treatment of patients after stent implantation or balloon dilatation and novel drug eluting stents**

(71) Applicant: Max-Planck-Gesellschaft zur Förderung der Wissenschaften e.V., 80539 München (DE)
(72) Inventor: Braun, Thomas, 61231 Bad Nauheim (DE); Boettger, Thomas, 61231 Bad Nauheim (DE)
(74) Representative: Vossius & Partner

(57) **Abstract**

The present invention relates to a nucleic acid molecule for use in the treatment or preventive treatment of a patient after stent implantation or balloon dilatation, wherein the nucleic acid molecule is selected from (a) a single-stranded nucleic acid molecule comprising or consisting of the sequence of SEQ ID NO: 1, 2, 3 or 4 or a sequence having at least 90% sequence identity to the sequence of SEQ ID NO: 1, 2, 3 or 4; (b) a hairpin RNA, wherein one of the regions forming the double-stranded portion of said hairpin RNA comprises or consists of the sequence of SEQ ID NO: 1, 2, 3 or 4 or a sequence having at least 90% sequence identity to the sequence of SEQ ID NO: 1, 2, 3 or 4; (c) an at least partially double-stranded RNA comprising two separate single strands, wherein a region within one of the strands, said region being located within the double-stranded portion of said double-stranded RNA, comprises or consists of the sequence of SEQ ID NO: 1, 2, 3 or 4 or a sequence having at least 90% sequence identity to the sequence of SEQ ID NO: 1, 2, 3 or 4; (d) a nucleic acid molecule encoding the nucleic acid molecule of (a) or the RNA (b); and (e) a nucleic acid molecule or a two nucleic acid molecules encoding the two separate single strands of the RNA of (c). The present invention also relates to a drug eluting stent comprising the nucleic acid molecule according to the invention.

SEQUENCE LISTING

<110> Max-Planck-Gesellschaft zur Förderung der Wissenschaften
e.V.

<120> Novel treatment of patients after stent implantation or balloon
dilatation and novel drug eluting stents

<130> P2943 EP

<160> 8

<170> PatentIn version 3.4

<210> 1
<211> 23
<212> RNA
<213> homo sapiens

<400> 1
guccaguuuu cccaggaauc ccu 23

<210> 2
<211> 21
<212> RNA
<213> homo sapiens

<400> 2
ugagaugaag cacuguagcu c 21

<210> 3
<211> 23
<212> RNA
<213> mus musculus

<400> 3
guccaguuuu cccaggaauc ccu 23

<210> 4
<211> 21
<212> RNA
<213> mus musculus

<400> 4
ugagaugaag cacuguagcu c 21

<210> 5
<211> 88
<212> RNA
<213> homo sapiens

<400> 5
caccuugucc ucacggucca guuuucccag gaaucccuua gaugcuaaga uggggauucc 60

uggaaauacu guucuugagg ucaugguu 88

<210> 6
<211> 106
<212> RNA
<213> homo sapiens

<400> 6
gcgcagcgcc cugucuccca gccugaggug cagugcugca ucucugguca guugggaguc 60

ugagaugaag cacuguagcu caggaagaga gaaguuguuc ugcagc 106

<210> 7
<211> 70
<212> RNA
<213> mus musculus

<400> 7
cucacggucc aguuuuccca ggaaucccuu ggaugcuaag auggggauuc cuggaaauac 60

uguucuugag 70

<210> 8
<211> 63
<212> RNA
<213> mus musculus

<400> 8
ccugaggugc agugcugcau cucuggucag uugggagucu gagaugaagc acuguagcuc 60

agg 63

## Description

The present invention relates to a nucleic acid molecule for use in the treatment or preventive treatment of a patient after stent implantation or balloon dilatation, wherein the nucleic acid molecule is selected from (a) a single-stranded nucleic acid molecule comprising or consisting of the sequence of SEQ ID NO: 1, 2, 3 or 4 or a sequence having at least 90% sequence identity to the sequence of SEQ ID NO: 1, 2, 3 or 4; (b) a hairpin RNA, wherein one of the regions forming the double-stranded portion of said hairpin RNA comprises or consists of the sequence of SEQ ID NO: 1, 2, 3 or 4 or a sequence having at least 90% sequence identity to the sequence of SEQ ID NO: 1, 2, 3 or 4; (c) an at least partially double-stranded RNA comprising two separate single strands, wherein a region within one of the strands, said region being located within the double-stranded portion of said double-stranded RNA, comprises or consists of the sequence of SEQ ID NO: 1, 2, 3 or 4 or a sequence having at least 90% sequence identity to the sequence of SEQ ID NO: 1, 2, 3 or 4; (d) a nucleic acid molecule encoding the nucleic acid molecule of (a) or the RNA (b); and (e) a nucleic acid molecule or a two nucleic acid molecules encoding the two separate single strands of the RNA of (c). The present invention also relates to a drug eluting stent comprising the nucleic acid molecule according to the invention.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The technical field of the invention is the treatment of patients after stent implantation or balloon dilatation. After such surgical interventions complications like restenosis, and/or thrombosis can occur.

The reason for the occurrence of restenosis after stent implantation or balloon dilatation is normally an increased proliferation of cells in the vessel wall. One of this proliferating cell types in the vessel wall (tunica) is vascular smooth muscle cells (VSCMs) found within the tunica media of of large and small arteries and veins. Proliferation of VSCMs stimulates in turn the proliferation of fibroblasts in the tunica. VSMCs are highly specialized cells, which regulate the lumenal diameter of small arteries/arterioles called resistance vessels thereby contributing significantly to the regulation of blood pressure. The tone of VSMCs is controlled by angiotensin II and adrenergic stimulation although numerous other signalling molecules affect the contractile status of VSMCs (1). VSMCs proliferate rarely under normal physiological conditions in adult tissues but will undergo major phenotypic changes from the contractile to the synthetic phenotype in response to environmental cues such as hypertension, vascular injury, and arteriosclerosis (2). Early atherosclerotic lesions are often characterized by focal accumulation of VSMCs within the intima (vessel wall). The exact function of VSMCs in atherosclerosis, however, is still a matter of debate (3). VSMCs might contribute to the development of atherosclerotic plaques by synthesizing pro-inflammatory mediators, vascular cell adhesion molecules, and matrix molecules required for the retention of lipoproteins (4). The ability for contraction, proliferation and secretion depends on the differentiation status of VSMC, which is affected by a large number of factors including mechanical forces, contractile agonists such as angiotensin II, extracellular matrix components, neuronal factors, reactive oxygen species, endothelial-VSMC interactions, thrombin, PDGF and TGF-1. Transcriptional responses to these factors are mostly mediated by SRF and its co-factor myocardin, which play a key role in the regulation of VSMC markers, as well as by members of the Kruppel-like transcription factor family of zinc finger proteins (reviewed in (1)). SRF-dependent gene transcription is controlled at various posttranscriptional levels including - among others - differential splicing, phosphorylation and RhoA/Rho kinase (ROK)-dependent SRF nuclear translocation and/or activation (reviewed in (1)). In summary, a lot of information has been compiled about VSCMs. This information, however, has not led to the development of means or methods to treat a patient after stent implantation or balloon dilatation.

Disturbances in the blood flow, like restenosis after stent implantation or balloon dilatation occurs in about 10%-30% of patients within 6 months after implantation of the stent. In patients with a repeated stent implantation or balloon dilatation the risk of restenosis is 30-60%. In risk patients, like diabetic patients or patients with small or elongated vessel, restenosis after stent implantation or balloon dilatation occurs in up to 70% of patients. Therefore, restenosis after stent implantation or balloon dilation is also called the Achilles' heel of stent implantation and balloon dilatation. Although stent implantation and balloon dilatation are successful surgical interventions they often cannot prevent further surgical steps like a bypass operation. Therefore, presently many research projects seek to find substances to prevent restenosis after stent implantation or balloon dilatation. The presently used or developed substances are substances which inhibit the proliferation of cells, e.g. via inhibiting degradation of microtubles or blocking cellular signal transduction pathways. Such cell proliferation inhibitor substances are also used to coat stents in order to provide drug eluting stents. In addition to the substances inhibiting cell proliferation, it is presently required to treat patients after stent implantation and balloon dilatation with inhibitors of blood coagulation for a long term. Such blood coagulation inhibitors, like Phenprocoumon (Marcumar) have severe side effects.

Therefore, there is a need to find novel means and methods for use in the treatment of patients after stent implantation and balloon dilatation. This need is addressed by the present invention.

Accordingly, the present invention relates to a nucleic acid molecule for use in the treatment or preventive treatment of a patient after stent implantation or balloon dilatation, wherein the nucleic acid molecule is selected from
(a) a single-stranded nucleic acid molecule comprising or consisting of the sequence of SEQ ID NO: 1, 2, 3 or 4 or a sequence having at least 90% sequence identity to the sequence of SEQ ID NO: 1, 2, 3 or 4;
(b) a hairpin RNA, wherein one of the regions forming the double-stranded portion of said hairpin RNA comprises or consists of the sequence of SEQ ID NO: 1, 2, 3 or 4 or a sequence having at least 90% sequence identity to the sequence of SEQ ID NO: 1, 2, 3 or 4;
(c) an at least partially double-stranded RNA comprising two separate single strands, wherein a region within one of the strands, said region being located within the double-stranded portion of said double-stranded RNA, comprises or consists of the sequence of SEQ ID NO: 1, 2, 3 or 4 or a sequence having at least 90% sequence identity to the sequence of SEQ ID NO: 1, 2, 3 or 4;
(d) a nucleic acid molecule encoding the nucleic acid molecule of (a) or the RNA (b); and
(e) a nucleic acid molecule or a two nucleic acid molecules encoding the two separate single strands of the RNA of (c).

The term "nucleic acid molecule", in accordance with the present invention, includes DNA, such as cDNA, antisense DNA or genomic DNA, and RNA. It is understood that the term "RNA" as used herein comprises all forms of RNA including mRNA, miRNA (micro RNA), hnRNA, siRNA and asRNA (antisense). Preferably the term "nucleic acid molecule" is an RNA. More preferably the nucleic acid molecule is a single stranded RNA, hnRNA (hairpin RNA) or an at least partially double stranded RNA. The nucleic acid sequence may also comprise regulatory regions or other untranslated regions. The nucleic acid molecule is most preferably a miRNA. Where the nucleic acid molecule od (d) or (e) is a nucleic acid molecule encoding an RNA or another nucleic acid molecule, said nucleic acid molecule of (d) or (e) is usually a DNA molecule such as cDNA or genomic DNA, and the enclosed nucleic acid molecule is usually an RNA. The term "nucleic acid molecule" is interchangeably used in accordance with the invention with the term "polynucleotide".

The term "siRNA" in accordance with the present invention refers to small interfering RNA, also known as short interfering RNA or silencing RNA. siRNAs are a class of 18 to 30, preferably 20 to 25, most preferred 21 to 23 or 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome. siRNAs have a well defined structure: a short double-strand of RNA (dsRNA), advantageously with at least one RNA strand having an overhang. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Thus, any gene of which the sequence is known can in principle be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Also preferably at least one RNA strand has a 5'- and/or 3'-overhang. Preferably, one or both ends of the double-strand has a 3'-overhang from 1-5 nucleotides, more preferably from 1-3 nucleotides and most preferably 2 nucleotides. In general any RNA molecule suitable to act as siRNA is envisioned in the present invention. The most efficient silencing was so far obtained with siRNA duplexes composed of 21-nt sense and 21-nt antisense strands, paired in a manner to have 2-nt 3'- overhangs. The sequence of the 2-nt 3' overhang makes a small contribution to the specificity of target recognition restricted to the unpaired nucleotide adjacent to the first base pair (Elbashir et al. Nature. 2001 May 24;411(6836):494-8). 2'-deoxynucleotides in the 3' overhangs are as efficient as ribonucleotides, but are often cheaper to synthesize and probably more nuclease resistant. A "miRNA" in accordance with the present invention is a micro RNA, which is a single-stranded RNA molecule regulating gene expression. The term "miRNA" includes pri-miRNA, pre-miRNA and mature miRNA. miRNAs are encoded by genes from whose DNA they are transcribed but miRNAs are not translated into protein (i.e. they are non-coding RNAs); instead each primary transcript (a pri-miRNA) is processed into a short stem-loop structure called a pre-miRNA, then in a miRNA duplex (an at least partially double stranded miRNA of preferably 21-23nt) and finally into a functional mature miRNA. Mature miRNA molecules are single-stranded and partially complementary to one or more messenger RNA (mRNA) molecules, and their main function is to down-regulate gene expression. The genes encoding miRNAs are generally much longer than the processed mature miRNA molecule; miRNAs are first transcribed as primary transcripts or pri-miRNA with a cap and poly-A tail and processed to short, usually about 70-nucleotide stem-loop structures known as pre-miRNA in the cell nucleus. This processing is performed in animals by a protein complex known as the Microprocessor complex, consisting of the nuclease Drosha and the double-stranded RNA binding protein Pasha. These pre-miRNAs are then processed to mature miRNAs (normally 21 to 23 nucleotides in length) in the cytoplasm by interaction with the endonuclease Dicer, which also initiates the formation of the RNA-induced silencing complex (RISC). This complex is responsible for the gene silencing observed due to miRNA expression and RNA interference.

A "shRNA" in accordance with the present invention is a short hairpin RNA, which is a sequence of RNA that makes a (tight) hairpin turn that can also be used to silence gene expression via RNA interference. shRNA preferably utilizes the U6 promoter for its expression. The shRNA hairpin structure is cleaved by the cellular machinery into siRNA, which is then bound to the RNA-induced silencing complex (RISC). This complex binds to and cleaves mRNAs which match the shRNA that is bound to it.

In those embodiments where the nucleic acid molecule of the invention comprises (rather than consists of) the recited sequence, additional nucleotides extend over the specific sequence either on the 5' end or the 3' end or both. Those additional polynucleotides may be of heterologous or preferably homologous nature and may comprise stretches of about 3 to 100 nucleotides although higher or lower values are not excluded.

The nucleic acid molecule as defined in (a) supra include single-stranded RNA molecules such as mature miRNA as well as antisense DNA. Hence, the nucleic acid molecule of embodiment (a) may be DNA, RNA, or a nucleic acid comprising both ribonuleotides and desoxyribonuleotides. It is preferred that the nucleic acid molecule of (a) is human or mouse mature miR-145 as shown in SEQ ID Nos. 1 and 3 or human or mouse mature miR-143 as shown SEQ ID Nos. 2 and 4. The hairpin RNA as defined in (b) supra is includes pre-miRNA and pri-miRNA. It is preferred that the pri-mRNA or pre-miRNA is the pri-mRNA or pre-miRNA consisting of or comprising the SEQ ID Nos. 5, 6, 7 or 8 or comprising SEQID Nos. 1, 2, 3, or 4. The at least in part double-stranded RNA as defined in (c) supra includes RNAi agents such as siRNAs and miRNA duplexes.

The nuclei acid molecules according to the invention, may also comprise chemically modified nucleotides. Modifications of nucleotides acids are well known to person skilled in the art and include locked nucleic acids. Envisaged are modified internucleotide linkages such as phosphorothioates.

In RNAs according to the invention one or more nucleotides, but less than 50% of the total number of nucleotides may be replaced with their counterparts.

As mentioned, also encompassed by the present invention are nucleic acid molecules comprising or consisting of a sequence having at least 90% identity (such as at least 92.5%, more preferred at least 95%, even more preferred at least 97,5%, and most preferred 100% identity) at the nucleic acid level with the sequence of SEQ ID NOs: 1, 2, 3, or 4 over the entire length of SEQ ID NOs: 1, 2, 3, or 4. It is to be understood that the comparison or alignment of sequences referred to herein means an alignment with the nucleotide sequence. In one embodiment, the two sequences, when aligned, display the at least 90% identity over the same length, i.e. without either sequence extending 3'or 5' over the other sequence. Those having skill in the art will know how to determine percent sequence identity between/among sequences using, for example, algorithms such as those based on CLUSTALW computer programme (Thompson Nucl. Acids Res. 2 (1994), 4673-4680) or FASTA (Pearson and Lipman, Proc. Natl. Acad. Sci., 1988, 85; 2444), as known in the art. Although the FASTA algorithm typically does not consider internal non-matching deletions or additions in sequences, i.e., gaps, in its calculation, this can be corrected manually to avoid an overestimation of the % sequence identity. CLUSTALW, however, does take sequence gaps into account in its identity calculations. Also available to those having skill in this art are the BLAST and BLAST 2.0 algorithms (Altschul, Nucl. Acids Res., 1977, 25:3389). The BLASTN programme for nucleic acid sequences uses as default a word length (W) of 11, an expectation (E) of 10, M=5, N=4, and a comparison of both strands. The BLOSUM62 scoring matrix (Henikoff, Proc. Natl. Acad. Sci., 1989, 89:10915) uses alignments (B) of 50, expectation (E) of 10, M=5, N=4, and a comparison of both strands. All those programmes may be used for the purposes of the present invention. However, preferably the BLAST programme is used. Accordingly, all the nucleic acid molecules having the prescribed function and further having a sequence identity of at least 90% as determined with any of the above recited or further programmes available to the skilled person and preferably with the BLAST programme fall under the scope of the invention.

The term "patient" in accordance with the invention is a vertebrate. It is preferred that the vertebrate is a mammal. It is even more preferred that the mammal is a domestic mammal or primate. It is mostly preferred that the patient is a mouse or human.

The term "stent" in accordance with the present invention refers to is a man-made 'tube' inserted into a natural passage/conduit in the body to prevent, or counteract, a disease-induced, localized flow constriction. Stents are used in a variety of vessels aside from the coronary arteries also as a component of peripheral artery angioplasty. Stents are preferably coronary stents. A coronary stent is a stent placed in a coronary artery to treat coronary heart disease, for example as part of a procedure called percutaneous coronary intervention (PCI). Similar stents and procedures are used in non-coronary vessels, e.g., in the legs in peripheral artery disease. A stent is composed of special fabric supported by a rigid structure, usually metal. An stent on its own has no covering, and therefore is usually just a metal mesh. Although there are many types of stent, these stents are used mainly for vascular intervention. The device is used primarily in endovascular surgery. Stent are used to support weak points in arteries, such a point commonly known as an aneurysm. Stent are most commonly used in the repair of an abdominal aortic aneurysm, in a procedure called an EVAR. One theory behind the procedure is that once in place inside the aorta, the stent acts as a false lumen for blood to travel through, instead of flowing into the aneurysm sack.

The term "balloon dilatation" in accordance with the invention refers to a method to widen narrowed blood Vessels. The method comprises tightly folded balloons to be passed into the narrowed locations and then inflated to a fixed size using water pressures some 75 to 500 times normal blood pressure (6 to 20 atmospheres). Peripheral balloon dilatation refers to the use of mechanical widening in opening blood vessels other than the coronary arteries. It is often called percutaneous transluminal angioplasty or PTA for short. PTA is most commonly done to treat narrowings in the leg arteries, especially the common iliac, external iliac, superficial femoral and popliteal arteries. PTA can also be done to treat narrowings in veins, etc. It is preferred that the balloon dilatation is a coronary balloon dilatation, which is also know as percutaneous coronary intervention (PCI) or coronary angioplasty. Coronary balloon dilatation is a therapeutic procedure to treat the stenotic (narrowed) coronary arteries of the heart found in coronary heart disease.

In recent years it has been revealed that posttranscriptional regulation of gene expression critically depends on miRNAs, a class of approximately 22 nucleotide non-coding RNAs, which repress protein expression of target mRNAs by mRNA degradation or translational repression (5). Using miRNA target prediction algorithms hundreds of potential target mRNAs for a specific miRNA can be identified and miRNA target binding sites have been predicted in almost any transcript. Many miRNAs are expressed in a tissue-specific manner and play pivotal roles in the control of proliferation and differentiation of different cell types (6-9). According to the invention, to understand the role of miRNAs in the control of VSMC differentiation and function the presence of miRNAs in organs with a high content of SMCs using miRNA microarray hybridization was screened. It has been found by the inventors that the expression of the miR-143/145 cluster becomes confined to smooth muscle cells during embryonic development. SEQ ID No. 1 and SEQ ID No. 3 shows the human and mouse mature microRNA miR-145, respectively. SEQ ID No. 2 and SEQ ID No. 4 shows the human and mouse mature micro RNA miR-143, respectively. According to the invention, the miR-143/145 cluster is instrumental to acquire and/or maintain the contractile phenotype of VSMCs and controls the concentration of proteins, which regulate contractility of the VSMCs. Loss of the miRNA cluster assumingly results in deregulated blood pressure and formation of neointimal lesions in normolipidemic mice. The present invention defines the miR-143/145 gene cluster as a major regulator of the contractile phenotype of VSMCs. It is demonstrated that the miR-143/145 gene cluster governs the expression level of molecules, which control the balance between the synthetic (proliferating) and the contractile (nonproliferating) state of smooth muscle cells and directly affect contractility of VSMCs. The restricted expression of miR-143/145 initially in early cardiomyocytes and then in smooth muscle cells identifies this miRNA family as a cell type-specific regulator of smooth muscle cells, which enlarges the relatively small group of smooth muscle cell specific regulators. The microarray and reporter-gene based expression analysis according to the examples of the invention did not reveal major expression sites of miR-143 and miR-145 in other cell types although low level or context-dependent expression patterns cannot be ruled out. Defects during early embryogenesis in miR-145 knock-out mice were not observed, which is in contrast to the recently acclaimed function of miR-145 as an inducer of lineage-restricted differentiation (28). Thus, the role of miR-143/145 identified according to the invention allows to use a nucleic acid molecule as defined by the invention for the purpose according to the invention. The use of the nucleic acid molecule of the invention allows for the specific inhibition of the proliferation of VSMCs and thus for the specific treatment or preventive treatment of patients after stent implantation or balloon dilatation. As described herein above, methods for inhibiting restenosis known in the state of the art are based on inhibiting cell proliferation of any cell type, mostly dependent on the proliferation rate. Moreover, the influence of miR-143/145 on blood pressure may allow for omitting or at least decrease the treatment of patients with blood coagulation after stent implantation or balloon dilatation.

A preferred embodiment of the invention relates to the nucleic acid molecule as defined in accordance with the invention for use in the preventive treatment of a patient after stent implantation or balloon dilatation, wherein the treatment is for the prevention of restenosis.

"Restenosis" in accordance with the invention refers to renarrowing of a blood vessel after stent implantation or balloon dilatation in said blood vessel. The renarrowing results in response to the vascular injury that takes place during interventional procedure. As explained above, restenosis occurs in about 10-30% of patients after stent implantation. Generally restenosis is caused by hyperproliferation of cell in the blood vessel wall, which inhibits the blood flow.

Another preferred embodiment of the invention relates to the nucleic acid molecule as defined in accordance with the invention for use in the treatment or preventive treatment of a patient after stent implantation or balloon dilatation, wherein said treatment or preventive treatment is for the prevention or treatment of thrombosis.

"Thrombosis " in accordance with the invention is the formation of a blood clot (thrombus) inside a blood vessel, obstructing the flow of blood through the circulatory system. When a blood vessel is injured, the body uses platelets and fibrin to form a blood clot, because the first step in repairing it (hemostasis) is to prevent loss of blood. If that mechanism causes too much clotting, and the clot breaks free, an embolus is formed. When a thrombus occupies more than 75% of surface area of the lumen of an artery, blood flow to the tissue supplied is reduced enough to cause symptoms because of decreased oxygen (hypoxia) and accumulation of metabolic products like lactic acid. More than 90% of obstruction can result in anoxia, the complete deprivation of oxygen, and infarction, a mode of cell death.

An even other preferred embodiment of the invention relates to the nucleic acid molecule as defined in accordance with the invention for use in the treatment or preventive treatment of a patient after stent implantation or balloon dilatation, wherein said treatment or preventive treatment is for the prevention or treatment of hypertension.

"Hypertension" (also referred to as high blood pressure) in accordance with the invention is a medical condition in which the blood pressure is chronically elevated. Hypertension can be further classified with increasing preference as prehypertension (diastolic blood pressure 120-139 mmHg and and/or diastolic blood pressure 80-89 mmHg), stage 1 hypertension (diastolic blood pressure 140-159 mmHg and and/or diastolic blood pressure 90-99 mmHg) and stage 2 hypertension (diastolic blood pressure at least 160 mmHg and and/or diastolic blood pressure at least 100 mmHg). Methods of quantifying the blood pressure are well know to the skilled person. Blood pressure in accordance with the invention is the pressure (force per unit area) exerted by circulating blood on the walls of blood vessels, and constitutes one of the principal vital signs. The pressure of the circulating blood decreases as it moves away from the heart through arteries and capillaries, and toward the heart through veins. When unqualified, the term blood pressure usually refers to brachial arterial pressure: that is, in the major blood vessel of the upper left or right arm that takes blood away from the heart. Blood pressure may, however, sometimes be measured at other sites in the body, for instance at the ankle. The ratio of the blood pressure measured in the main artery at the ankle to the brachial blood pressure gives the Ankle Brachial Pressure Index (ABPI).Normal systolic blood pressure is 90-119 mmHg and normal diastolic blood pressure is 60-79 mmHg.

In accordance with the invention the nucleic acid molecule of the invention may be modified. Means and methods for such modifications are described in Soutschek et al. 2005 (Nature 432, 173-178). Such modified nucleic acid molecules can be prepared by stepwise solid-phase synthesis. In some cases, it may be desirable to add additional chemical moieties to the nucleic acid molecule of the invention, e.g. to enhance pharmacokinetics of the compound. Such a moiety may be covalently attached, typically to a terminus of the nucleic acid molecule, according to standard synthetic methods. For example, addition of a polyethyleneglycol moiety or other hydrophilic polymer, e.g., one having 10-100 monomeric subunits, may be useful in enhancing solubility. One or more charged groups, e.g., anionic charged groups such as an organic acid, may enhance cellular uptake. In selecting a moiety for attachment or modification of an antisense oligomer, it is generally desirable to select chemical compounds of groups that are biocompatible and likely to be tolerated by a subject without undesirable side effects.

In another preferred embodiment of the invention, the nucleic acid molecule as defined in accordance with the invention is fused to a lipid.

In an even more preferred embodiment of the invention, the lipid fused to the nucleic acid molecule as defined in accordance with the invention is a cholesterol.

Means and methods to introduce lipid modifications and in particular a cholesterol modification to a nucleic acid molecule are described in Krützfeldt et al. 2005 (Nature 438, 685-689). Accordingly, a cholesterol may be linked through a hydroxylprolinol linkage to a nucleic acid molecule. Such modifications increase the efficiency of the uptake of a nucleic acid molecule and in particular of small RNAs, like miRNAs into the cell.

It is also preferred that the nucleic acid molecule according to the invention is present in a pharmaceutical composition. The pharmaceutical composition may, just as the nucleic acid molecule, be used in the treatment of a patient after stent implantation or balloon dilatation, wherein the treatment preferably prevents restenosis or thrombosis and prevents or treats hypertension.

The pharmaceutical composition prepared in accordance with the invention comprises a nucleic acid molecule as defined above, preferably a RNA like a antisense RNA, siRNA, shRNA or miRNA. Even more preferred the pharmaceutical composition may comprise a miRNA. The ability of antisense molecules, siRNA, shRNA and miRNA to potently, but reversibly, silence genes *in vivo* makes these molecules particularly well suited for use in the mentioned pharmaceutical composition. Ways of administering siRNA to humans are described in De Fougerolles et al., Current Opinion in Pharmacology, 2008, 8:280-285. Such ways are also suitable for other administering other small RNA molecules like shRNA or miRNA. Accordingly, such pharmaceutical compositions may be administered directly formulated as a saline, via liposome based and polymer-based nanoparticle approaches, as conjugated or complexation pharmaceutical compositions, or via viral delivery systems. Direct administration comprises injection into tissue, intranasal and intratracheal administration. Liposome based and polymer-based nanoparticle approaches comprise the cationic lipid Genzyme Lipid (GL) 67, cationic liposomes, chitosan nanoparticles and cationic cell penetrating peptides (CPPs). Conjugated or complexation pharmaceutical compositions comprise PEI-complexed antisense molecules, siRNA, The pharmaceutical composition is preferably administered to mammals such as domestic and pet animals. Most preferred it is administered to humans. The pharmaceutical compositions described herein can be administered to the subject at a suitable dose.

The pharmaceutical composition for use in accordance with the present invention can be formulated in conventional manner according to methods found in the art, using one or more physiological carriers or excipient, see, for example Ansel et al., "Pharmaceutical Dosage Forms and Drug Delivery Systems", 7th edition, Lippincott Williams & Wilkins Publishers, 1999. The pharmaceutical composition may, accordingly, be administered orally, parenterally, such as subcutaneously, intravenously, intramuscularly, intraperitoneally, intrathecally, transdermally, transmucosally, subdurally, locally or topically via iontopheresis, sublingually, by inhalation spray, aerosol or rectally and the like in dosage unit formulations optionally comprising conventional pharmaceutically acceptable excipients.

The pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

The pharmaceutical composition can be administered as sole active agent or can be administered in combination with other agents. It is preferred that such other agents are inhibitors of cell proliferation. Such inhibitors comprise but are not limited to inhibitors of microtuble degradation (e.g. Taxol) and inhibitors of cellular signalling (e.g. Rapamycin).

A further embodiment of the invention, refers to a drug eluting stent comprising a nucleic acid molecule as defined in accordance with the present invention.

According to this embodiment of the invention, the nucleic acid molecule of the invention or the pharmaceutical composition comprising the nucleic acid molecule of the invention may be delivered using a drug eluting stent, which is covered (the term "covered" encompasses the terms "attached, linked, coated and the like") by the nucleic acid molecule of the invention or the pharmaceutical composition. Means and methods for producing drug eluting stents are described in Udipi et al. 2007 (Journal of Biochemical Materials Research Part A, p. 1065-1071). In this regard, the nucleic acid molecule of the invention may be delivered by way of diffusion, or more preferably, by degradation of a polymeric matrix covering the drug eluting stent. Exemplary synthetic polymers which can be used to form the biodegradable delivery system covering a drug eluting stent include: polyamides, polycarbonates, polyalkylenes, polyalkylene glycols, polyalkylene oxides, polyalkylene terepthalates, polyvinyl alcohols, polyvinyl ethers, polyvinyl esters, poly-vinyl halides, polyvinylpyrrolidone, polyglycolides, polysiloxanes, polyurethanes and co-polymers thereof, alkyl cellulose, hydroxyalkyl celluloses, cellulose ethers, cellulose esters, nitro celluloses, polymers of acrylic and methacrylic esters, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), pory(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), polyvinyl acetate, poly vinyl chloride, polystyrene, polyvinylpyrrolidone, and polymers of lactic acid and glycolic acid, polyanhydrides, poly(ortho)esters, poly(butic acid), poly(valeric acid), and poly(lactide-cocaprolactone), and natural polymers such as alginate and other polysaccharides including dextran and cellulose, collagen, chemical derivatives thereof (substitutions, additions of chemical groups, for example, alkyl, alkylene, hydroxylations, oxidations, and other modifications routinely made by those skilled in the art), albumin and other hydrophilic proteins, zein and other prolamines and hydrophobic proteins, copolymers and mixtures thereof. In general, these materials degrade either by enzymatic hydrolysis or exposure to water in vivo, by surface or bulk erosion. Examples of nonbiodegradable polymers suitable for covering a drug eluting stents include ethylene vinyl acetate, poly(meth)acrylic acid, polyamides, copolymers and mixtures thereof. Bioadhesive polymers of particular interest include bioerodible hydrogels described by H. S. Sawhney, C. P. Pathak and J. A. Hubell in Macromolecules, (1993) 26:581-587, the teachings of which are incorporated herein, polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate). In certain embodiments of the invention, the administration of the nucleic acid molecule of the invention maybe designed so as to result in sequential exposures to the composition over a certain time period, for example, hours, days, weeks, months or years. This may be accomplished, for example, by a sustained or controlled release delivery system covering the drug eluting stent in which the composition is delivered over a prolonged period without repeated administrations. Maintaining a constant concentration of the composition may be preferred in some cases. Other drug eluting stents suitable for use with the present invention include time-release, delayed release, sustained release, or controlled release of the nucleic acid according to the invention. Such systems may avoid repeated administrations in many cases, increasing convenience to the subject and the physician. Many types of release delivery systems suitable for covering drug eluting stents are available and known to those of ordinary skill in the art. They include, for example, polymer-based systems such as polylactic and/or polyglycolic acids, polyanhydrides, polycaprolactones, copolyoxalates, polyesteramides, polyorthoesters, polyhydroxybutyric acid, and/or combinations of these. Microcapsules of the foregoing polymers containing drugs are described in, for example, U.S. Pat. No. 5,075,109. Other examples include nonpolymer systems that are lipid-based including sterols such as cholesterol, cholesterol esters, and fatty acids or neutral fats such as mono-, di- and triglycerides; hydrogel release systems. liposome-based systems; phospholipid based-systems; silastic systems; peptide based systems; wax coatings; compressed tablets using conventional binders and excipients; or partially fused implants. Specific examples include, but are not limited to, erosional systems in which the composition is contained in a form within a matrix (for example, as described in U.S. Pat. Nos. 4,452,775, 4,675,189, 5,736,152, 4,667,013, 4,748,034 and 5,239,660), or diffusional systems in which an active component controls the release rate (for example, as described in U.S. Pat. Nos. 3,832,253, 3,854,480, 5,133,974 and 5,407,686). The formulation may be as, for example, microspheres, hydrogels, polymeric reservoirs, cholesterol matrices, or polymeric systems. In some embodiments, the system may allow sustained or controlled release of the nucleic acid molecule of the invention to occur, for example, through control of the diffusion or erosion/degradation rate of the formulation containing the nucleic acid molecule of the invention. Examples of systems in which release occurs in bursts includes, e.g., systems in which the nucleic acid molecule of the invention is entrapped in liposomes which are encapsulated in a polymer matrix, the liposomes being sensitive to specific stimuli, e.g., temperature, pH, light or a degrading enzyme and systems in which the nucleic acid molecule of the inventuion is encapsulated by an ionically-coated microcapsule with a microcapsule core degrading enzyme. Examples of systems in which release of the nucleic acid molecule is gradual and continuous include, e.g., erosional systems in which the nucleic acid molecule is contained in a form within a matrix and effusional systems in which nucleic acid molecule permeates at a controlled rate, e.g., through a polymer. Such sustained release systems can be e.g., in the form of pellets, or capsules covering the drug eluting stents. Use of a long-term drug eluting stent may be particularly suitable in some embodiments of the invention. "Long-term release," as used herein, means that the stent covered by the nucleic acid molecule of the invention is constructed and arranged to deliver therapeutically effective levels of the nucleic acid molecule for at least 1 month or 2 months, and preferably at least 3 months or 4 months, or even longer in some cases. Long-term release formulations suitable to cover a drug eluting stent are well known to those of ordinary skill in the art, and include some of the release systems described above.

In another embodiment of the invention, the invention relates to an in vitro method of identifying an activator of expression of a microRNA, said microRNA comprising or consisting of the sequence of SEQ ID NO: 1, 2, 3 or 4 or a sequence having at least 90% sequence identity to the sequence of SEQ ID NO: 1, 2, 3 or 4, said method comprising:
(a) providing a cell or an in vitro expression system, said cell or system being capable of expressing said microRNA under the control of the native promoter;
(b) bringing into contact said cell or system with a test compound; and
(c) comparing the expression level of said microRNA after and prior to step (b), wherein an increase of the expression level is indicative of said compound being an activator of expression of said microRNA
wherein said activator is a pharmaceutically active agent for the treatment or preventive treatment of a patient after stent implantation or balloon dilatation, or a lead compound suitable for developing a pharmaceutically active agent for the treatment or preventive treatment of a patient after stent implantation or balloon dilatation.

The term "activator of expression of a microRNA" designates a compound which, in accordance with the present invention, specifically interacts with/specifically binds to the nucleic acid sequence of SEQ ID NOs: 1, 2, 3 or 4 including regulatory regions thereof and activates or increases the transcription of the target microRNA. It is preferred that the activator interacts with the native promoter of SEQ ID NOs: 1, 2, 3 or 4. Preferably, transcription is enhanced by at least 50%, more preferred at least 75% such as at least 90% or 95%, and even more preferred at least 98%.

In a preferred embodiment, the test compound is a small organic molecule. Small organic molecules are compounds of natural origin or chemically synthesized compounds. The term "small organic molecule" in accordance with the invention has the same meaning as in the art and preferably refers to molecules having a molecular weight less than 5000 g/mol, preferably less than 1000 g/mol, more preferably less than 750 g/mol and most preferably between 300 g/mol and 500 g/mol.

The efficiency of the activator compound can be quantified by methods comparing the level of activity in the presence of the activator to that in the absence of the activator. For example, as an activity measure may be used: the change in amount of microRNA formed, the change in the cellular phenotype and/or in the phenotype of an organism. Preferably, said method is effected in high-throughput format. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits the expected activity, said mixture of test compounds may be de-convoluted to identify the one or more test compounds in said mixture giving rise to said activity.

Moreover, it is preferred that the activator is a pharmaceutically active agent for the treatment of a patient after stent implantation or balloon dilatation, or a lead compound suitable for developing a pharmaceutically active agent for the treatment of a patient after stent implantation or balloon dilatation, wherein the treatment prevents restenosis and/or treats or prevents a thrombosis or hypertension after stent implantation or balloon dilatation.

According to this aspect of the invention, it is also preferred that the activator is used to cover a drug eluting stent, wherein the stent elutes the activator. Means and methods for a drug eluting stent are described herein above.

### THE FIGURES SHOW:

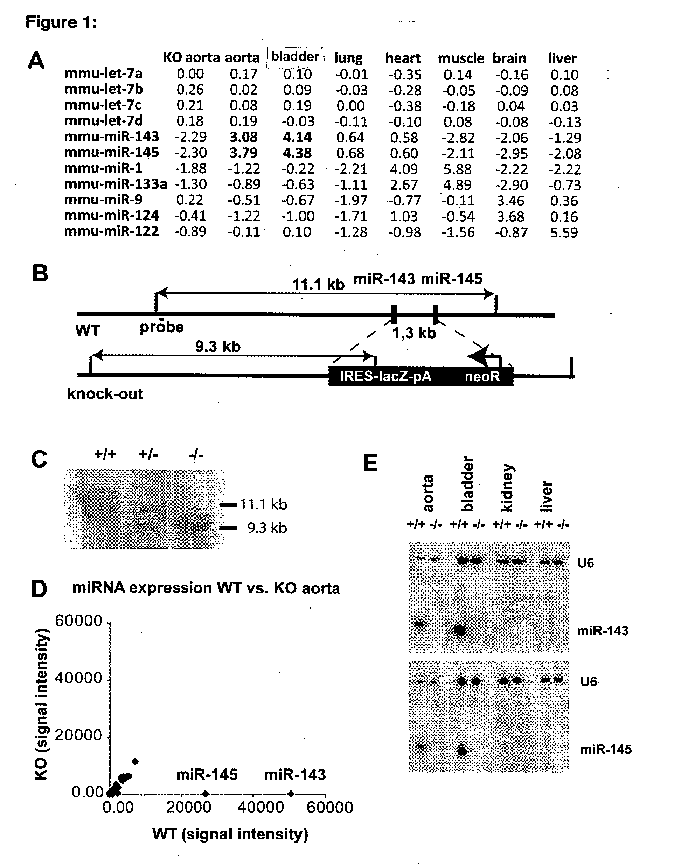

**Figure 1****: Microarray analysis of miR-143/145 expression and targeting strategy of the miR-143/145 cluster.** (A) miRNA microarray analysis of different mouse tissues. Expression values are ratios of tissue specific miRNA expression vs. reference miRNA from an E15.5 mouse embryo. Arrays were normalized to a ratio of 1 of the let-7a-g and let-7i signals; individual rows are centered. Both miRNAs show a similar expression profile in different organs. The miR-1/133a-cluster, miR-124/miR-9 and miR-122 are known markers of heart/muscle, brain and liver, respectively. (B) The miR-143 and miR-145 sequences are separated by a 1.3 kb fragment on mouse chromosome 18. Both miRNAs were deleted by insertion of an IRES-LacZ/neo^{R} cassette. EcoRV restriction fragments used for genotyping are indicated. (C) Southern blot analysis of wildtype, heterozygous and knockout animals using the 5' outside probe and EcoRV digested genomic DNA. (D) Microarray analysis of miRNA expression of a wildtype- vs. knockout-aorta. The diagram depicts the result of two microarray experiments with dye swapping for wild-type and knockout mice. The arrays were normalized to a ratio of medians of 1 for the let-7 signals and the result of the two experiments were combined by calculating the mean of signals after reversion of all ratios (1/x) of the dye swap control experiment. MiR-143 and miR-145 are shown in red, other miRNAs of the aorta are in blue. (E) Northern blot analysis of miRNA expression in wild-type and knockout tissues. Blots were probed with radioactively labelled U6/miR-143 and U6/miR- 145, respectively.

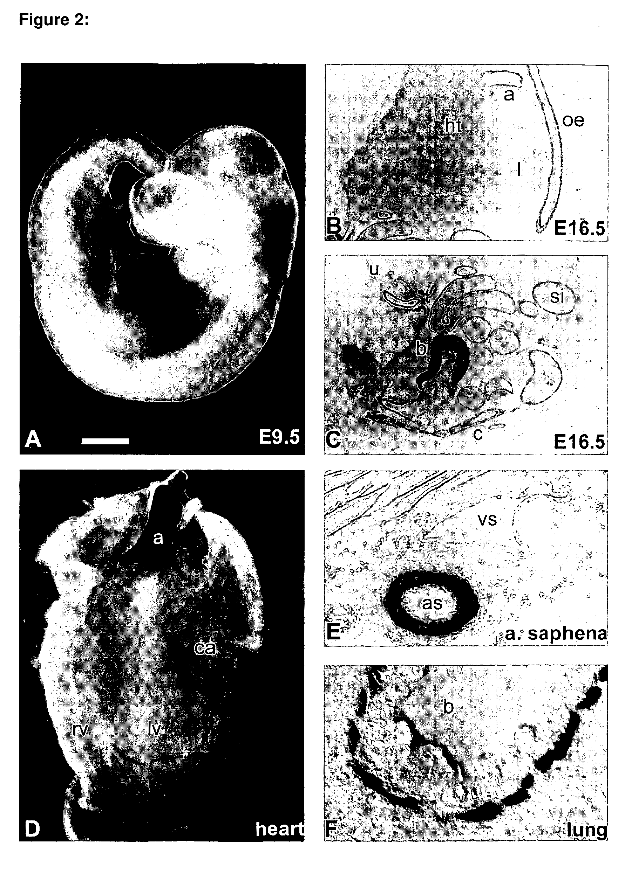

**Figure 2****: The miR-143/145 cluster is specifically expressed in the smooth muscle cell lineage.** (A-F) Specific expression of the lacZ reporter gene under control of the miR-143 promoter in smooth muscle cells. (A) miR-143/145 is initially expressed in the developing embryonic heart at E8.5 (not shown) to E9.5. (B, C) During fetal stages (E16.5) the 37 expression of the miR-143 reporter gene becomes confined to smooth muscle cells of the aorta (a), smaller blood vessels, oesophagus (oe), lung (I), small intestine (si), colon, (c) bladder (b), and umbilical cord (u). (D-F) In adult animals miR-143 expression is present in all smooth muscle cells throughout the body including the aorta (a), the coronary arteries (ca) of the cross sectioned heart (left ventricle-lv; right ventricle-rv), the saphenous artery (as) and the bronchi (b). Scale bar in (A) corresponds to (A): 0.6 mm; (B, C): 0.9 mm; (D): 1.2 mm; (E): 0.09 mm; (F): 0.06 mm.

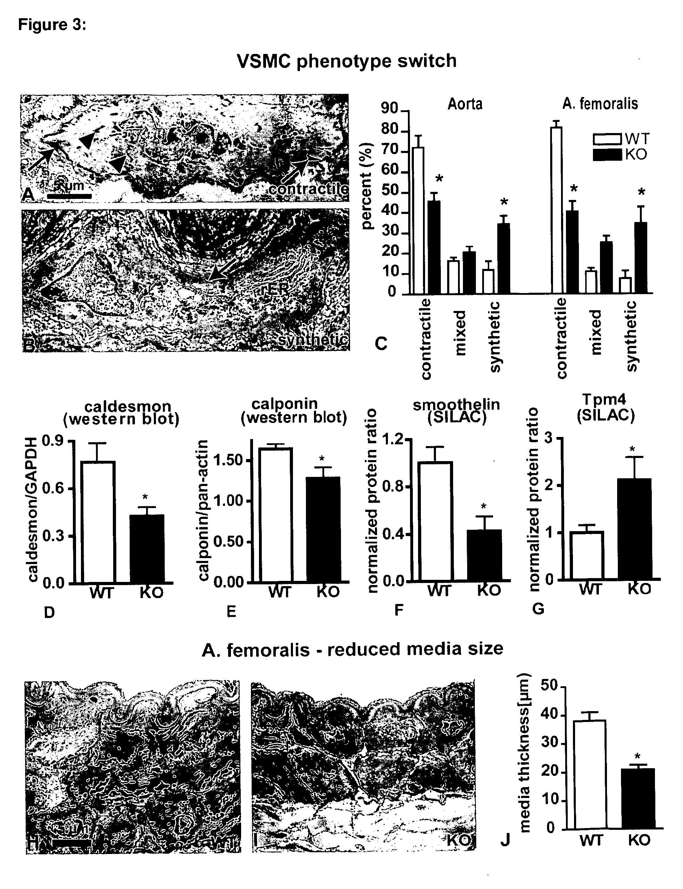

**Figure 3****: Smooth muscle cells of miR-143/145 knockout mice show a shift from a contractile to a synthetic phenotype and reduced media thickness.** (A, B) Ultrastructural features of contractile and synthetic SMC. Typical contractile SMCs (A) from WT animals show numerous focal adhesions (arrows) and intracellular dense bodies (arrowheads). In contrast, synthetic SMCs (B) from mutant mice only rarely display focal adhesions (arrow) and intracellular dense bodies and are rich in rough endoplasmatic reticulum (rER) (C) Relative numbers of synthetic and contractile VSMCs in aorta and A. femoralis. The number of synthetic smooth muscle cells is increased in the aorta and femoral arteries of knockout mice. (D-G) Quantification of markers of contractile SMCs (D-F) and synthetic SMCs (G) is depicted. (H, I) EM pictures of cross-sections through the A. femoralis. Note the presence of contractile and synthetic smooth muscle cells in WT and mutant vessel walls, respectively. (J) Quantification of media thicknesses. (*p<0.05, n=3/3, 300 cells counted/animal in (C); p<0.0001, n=8/7 in F; error bars: +SEM).

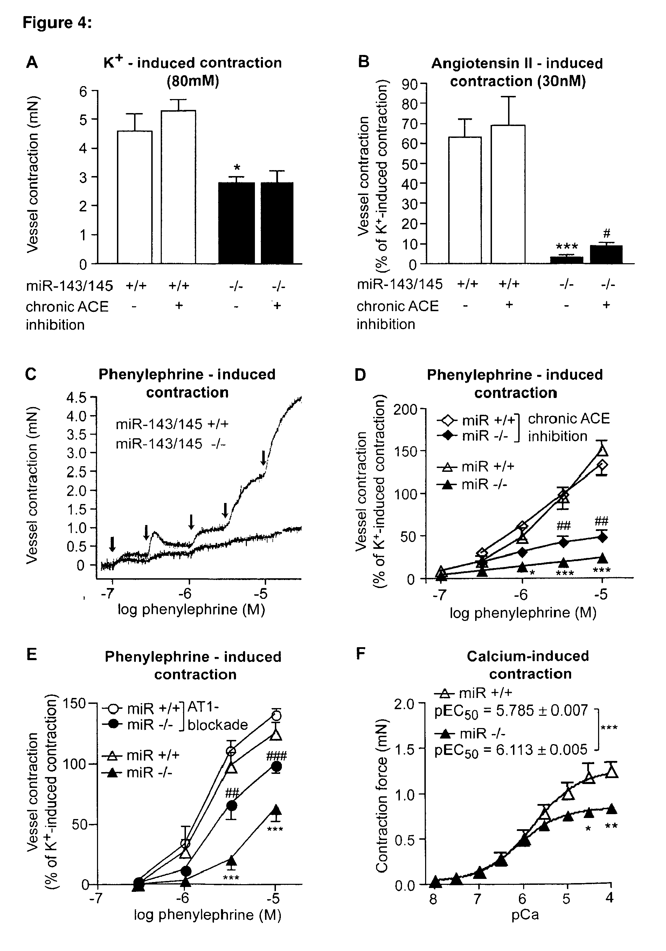

**Figure 4****: In vitro evaluation of contractile properties of isolated femoral artery rings mounted in a myograph.** (A) Vessel contraction induced by extracellular potassium was reduced to 61 % of the WT response (n=12/8 vessels WT/KO, *p<0.05). No change in contraction after potassiuminduced depolarization was observed after treatment with captopril (8/9 WT/KO vessels). (B) Vessel contraction induced by angiotensin II was significantly blunted in mutant arteries. Pretreatment with captopril significantly improved angiotensin II induced contractile responses (12/9 WT/KO vessels of untreated animals, ***p<0.0001 KO untreated vs. WT untreated; 8/9 WT/KO vessels of captopril-treated animals, #p<0.05 KO treated vs. KO untreated). (C, D) Isolated arteries stimulated with increasing concentrations of phenylephrine. Arrows indicate applications of phenylephrine. (D) Statistical analysis of phenylephrine and captopril responses. Captopril treatment improved responses of the KO vessels (12/8 WT/KO vessels of untreated animals, *p<0.05, ***p<0.001 KO untreated vs. WT untreated; 8/9 WT/KO vessels of captopril-treated animals, ##p<0.01 KO treated vs. KO untreated). (E) Contractile responses to phenylephrine stimulation of mesenteric arteries. Arterial rings from KO animals showed a significant enhancement in contractility after AT1 receptor blockade by losartan (7/8 WT/KO vessels from untreated animals, ***p<0.001 KO untreated vs. WT untreated; 10/8 WT/KO vessels from losartan-treated animals, ##p<0.01, ###p>0.001 KO treated vs. KO untreated). (F) pCa-force relationships in skinned femoral arteries. Maximal Ca2+ induced contraction was blunted in KO arteries and the pEC50 values were significantly shifted leftwards in KO skinned arteries (12/8 WT/KO vessels, **p<0.01, ***p<0.001 KO vs. WT); (error bars: +SEM).

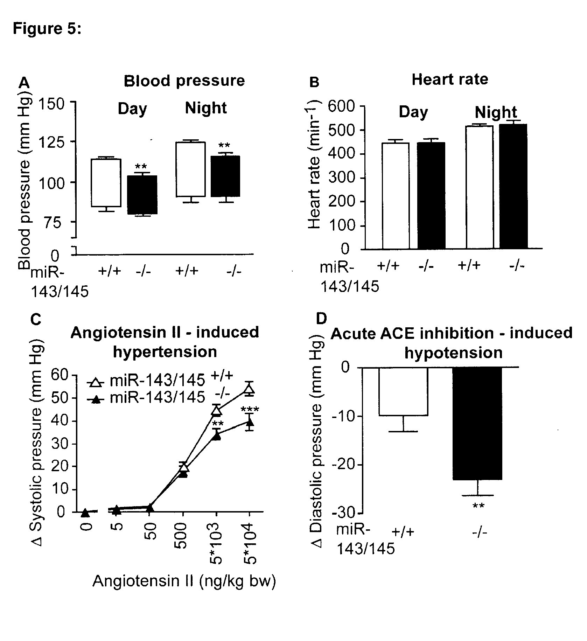

**Figure 5****: Hemodynamic measurements** (A, B) *In vivo* blood pressure and heart rate measurements by telemetry. (A) Boxes show blood pressure amplitudes with upper line representing systolic pressure, lower line diastolic pressure and respective error bars (mean ± SEM). Systolic blood pressure was significantly reduced in miR-143/145-deficient mice during day and nighttime periods (n=6/7 WT/KO, **p<0.01) while diastolic pressure did not differ significantly between genotypes. (B) No difference in heart rate was observed between genotypes. (C) During isoflurane anaesthesia, angiotensin II induced a larger increase in systolic blood pressure in wildtype than in KO (n=12/13 WT/KO, **p<0.01, ***p<0.001). (D) Acute inhibition of ACE by captopril lead to a stronger decrease in diastolic pressure in miR-143/145-deficient animals (n=11/13 WT/KO, **p<0.01); (error bars: +SEM).

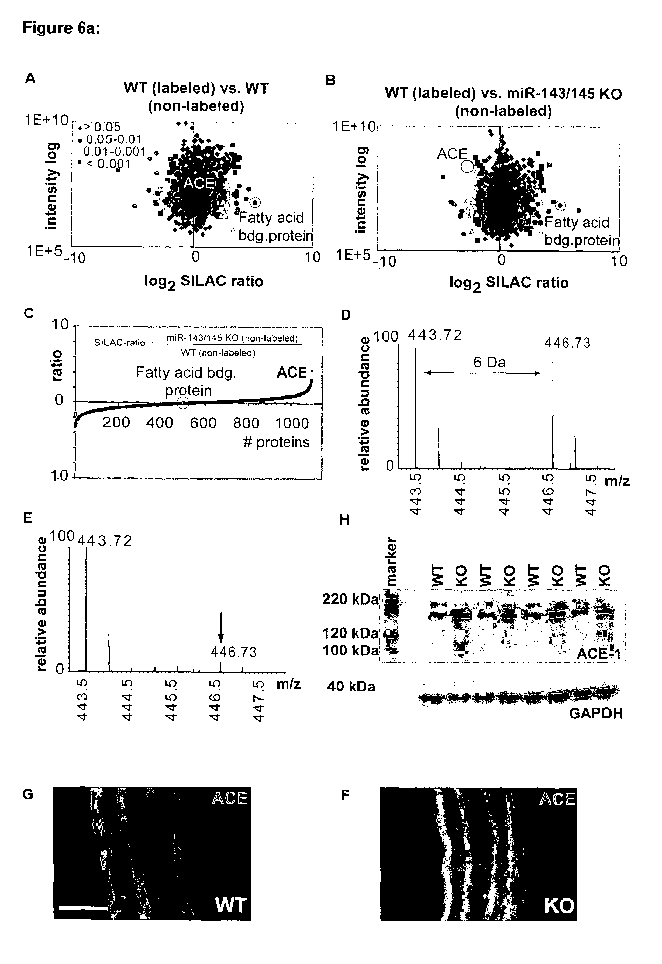

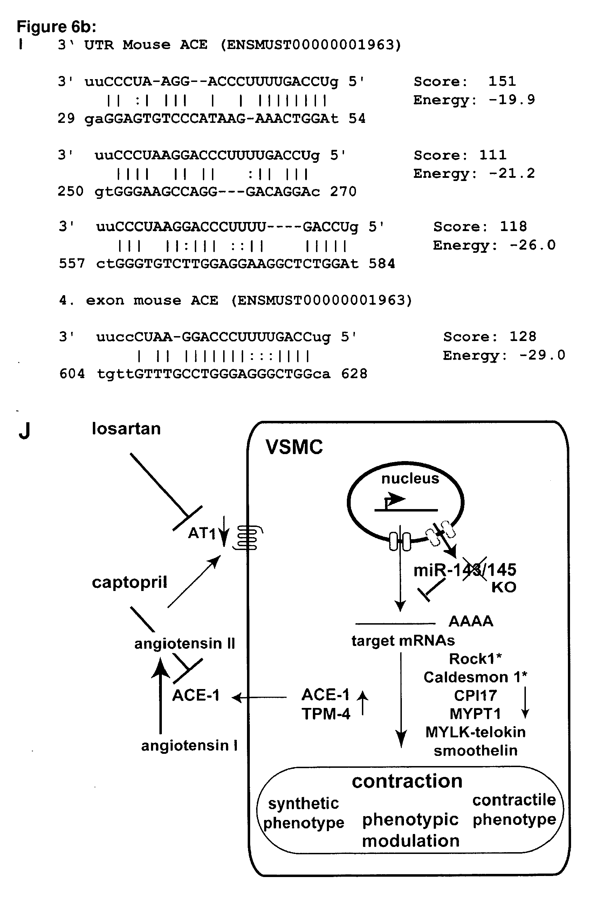

**Figure 6****: Angiotensin converting enzyme (ACE) is a target of miR-145.** Analysis of protein expression by SILAC-mouse based quantitative proteomics and western blot analysis. (A, B) SILAC-labelled (13C6Lys) wildtype aorta was mixed with non-labelled wild-type aorta (A) and miR-143/145 KO aorta (B). Protein ratios are plotted against added peptide intensities. Blue dots represent p-values above 0.05, red between 0.05 and 0.01, yellow between 0.01 and 0.001, and green below 0.001. (C) To obtain ratios between non-labelled wild-type and non-labelled miR-143/145 KO aortae both ratios from 13C6Lys - WT / WT (nonlabeled) and 13C6Lys - WT / miR-143/145 KO (non-labelled) were divided by each other. (D, E) Mass spectra of an ACE-1 peptide [YVEFSNK (MH2+)] SILAC pair with 13C6Lys - WT / WT (D) and a pair 13C6Lys - WT / miR-143/145 KO (E). Peaks with lower molecular weight correspond to the unlabeled peptide, peaks with higher molecular weight (plus 6 Da) correspond to the 13C6Lys - peptide. Calculation of ACE-1 signals from all samples revealed a 4.9 fold up-regulation of ACE-1 in KO vs. WT aortae. (n=3/3 animals, p<0.01; see Table 2). (G, F) Immunofluorescence analysis of ACE expression in cryosections of aorta (scale bar: 25 µm). (H) Western blot analysis of ACE-1 expression in WT and miR-143/145 KO aortae (n=4/4, p<0.001, ratio KO/WT: 4.3). (I) miR-145 binding sites in the 3'UTR and ORF of ACE predicted by the miRanda algorithm. (J) Schematic representation of the role of miR-143/145 in VSMCs.

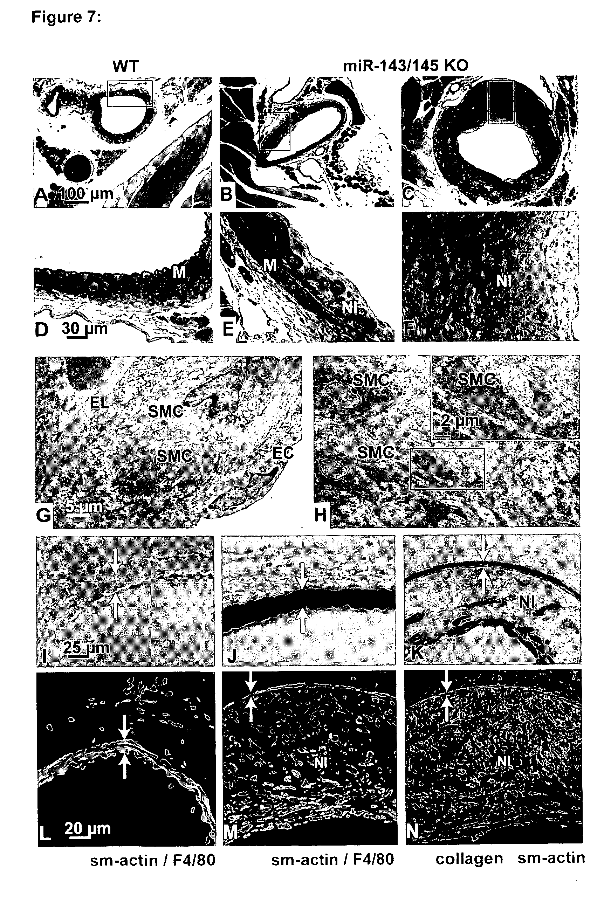

**Figure 7****: MiR-143/145 knockout mice develop neointimal lesions.** (A-C) Sections of WT (A) and KO (B, C) A. femoralis are shown; boxed regions are magnified below (D-F). Electron micrographs of early and late lesions are shown in G and H, respectively. (I-K) A. femoralis of WT (I), heterozygous (J) and homozygous animals (K) stained for β-galactosidase activity to indicate miR-143/145 locus activity. (L, M) Sections of WT (L) and KO vessels (M). Sections of the A.femoralis stained with antibodies against "- smooth muscle actin or the macrophage marker F8/40. (N) Sections of the A.femoralis stained with antibodies antibodies against "-smooth muscle actin and collagen I. Lesions were frequently found in KO animals but not in WT animals. (G) Formation of neointima with smooth muscle cells and macrophages (not shown) between the lamina elastica and endothelial cells. Lesions at later stages reached large volumes and showed a thinned media (I-N, arrows) but no constriction of the vessel. Please note the absence of foam cells or lipophilic inclusions (G, H), which was also indicated by the absence of Sudan IV staining (not shown). Smooth muscle cells were recognized by morphology (G, H), by anti-smooth muscle staining (M,N) or by the lacZ transgene expression under the control of the miR-143/145 locus (K). The plaques contain macrophages identified by the F4/80 marker (M) and deposits of amorphous collagen (N). Smooth muscle (G) and macrophages were also identified in early lesions (not shown). SMC smooth muscle cells, EL elastic lamina, NI neointima.

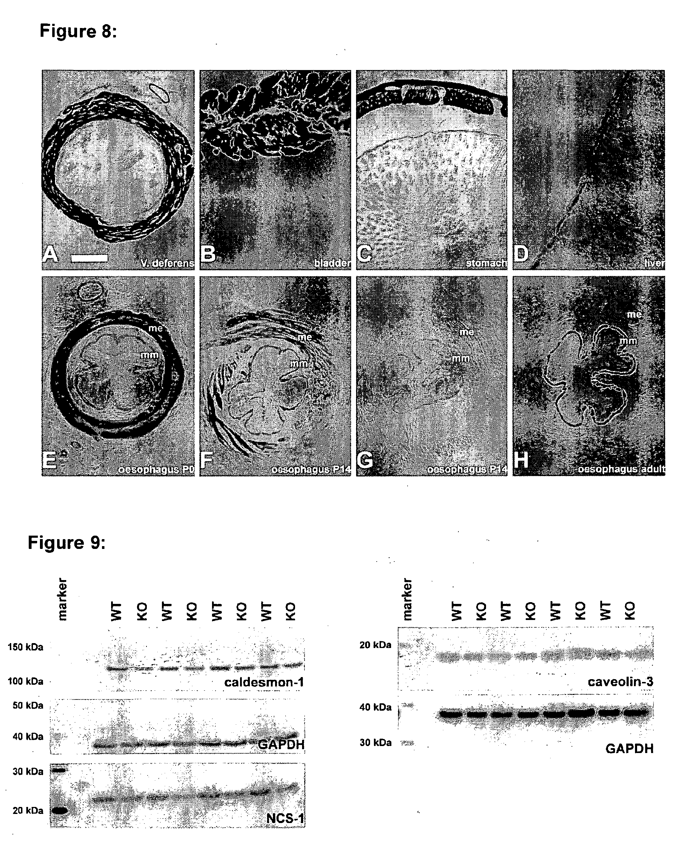

**Figure 8****: The miR-143/145 locus is active in smooth muscle cells of all SMC-containing organs.** Expression of the LacZ reporter, which indicates activity of the miR-143/145 locus, was detected in all parts of the smooth muscle coat of the Vas deferens (A), in the smooth muscle layers of the adult bladder (B), and in the stomach (C). In the liver (D), a signal was found in smooth muscle cells of the ramifications of the portal vein. The expression of the miR-143/145 reporter in the oesophagus (E-H) follows the described expression of other smooth muscle genes: At P0 (E) the reporter was expressed in the muscularis mucosae (mm) and the muscularis externa (me). At P14, expression in the esophagus was detected in the muscularis mucosa. Expression in the muscularis externa at this stage was only found proximal (F) but not distal to the stomach (G) since the distal part of the muscularis externa contains skeletal muscle cells at this stage. In the adult esophagus lacZ staining was only found in the muscularis mucosa (H). Scale bar corresponds to 189 µm in A, B, C, F, G, H, 46 µm in D, 94 µm in E.
**Figure 9****: The loss of mir-143/45 causes multiple secondary changes of protein expression.**Western blot analysis of caveolin-3, NCS-1 and caldesmon-1 expression in aortae of WT and miR-143/145 KO mice. Western blots were normalized to corresponding GAPDH signals. Please note the down-regulation of caveolin-3, NCS-1 and caldesmon-1 proteins, which were also found to be down-regulated in Affymetrix expression analysis. The GAPDH normalized ratio KO/WT was 0.7, 0.59 and 0.55 for caveolin-3, NCS-1 and caldesmon-1, respectively (n=4/4, p<0.05).

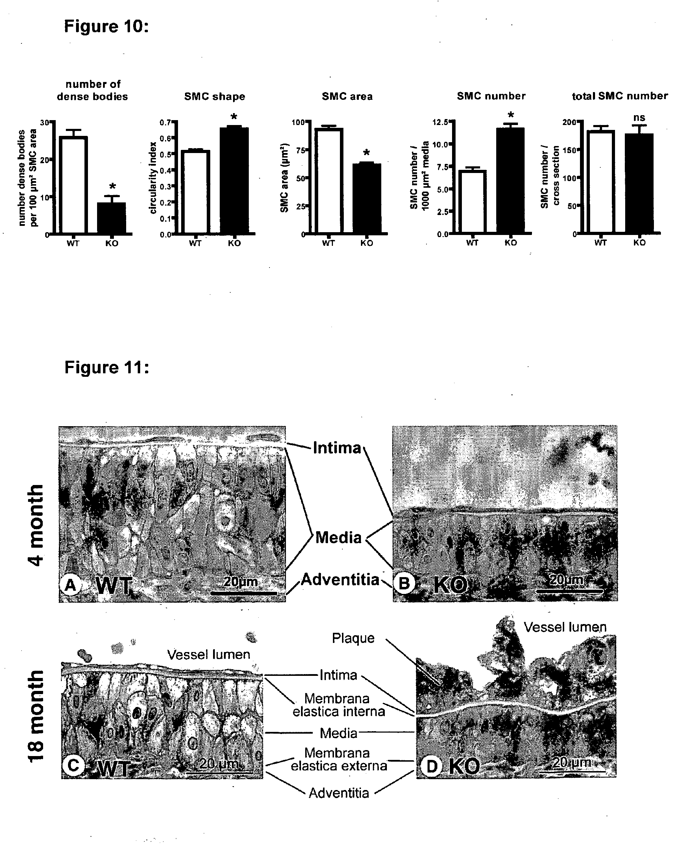

**Figure 10****: Changes in morphological parameters reflecting the phenotypic switch of VSMCs from contractile to synthetic.** Detailed morphometric analysis of VSMCs in the A. femoralis of KO and WT animals. A striking reduction in the number of dense bodies, which are essential to strengthen and support the contractile apparatus in VSMCs, was detected in KO animals (8.02 ± 2.19 in KO compared to 25.7 ± 2.13 in WT; mean ± SEM, n=3 for each genotype, p-value=0.0044 as determined by unpaired t-test). The VSMCs of KO mice were much more oval than those in WT (circularity index: 0.51 ± 0.01 in WT compared to 0.65 ± 0.02 in KO; mean ± SEM, n=3 for each genotype, p-value=0.0038 as determined by unpaired t-test). The shape of VSMCs (circularity index) was determined concomitantly with the area covered by VSMCs using the Image J program with at least 300 randomly selected cells per 3 transversally sectioned arterial segments (minimum 100 µm). The size of the VSMCs of the KO was reduced, the number of VSMC/area was increased, while the total cell number did not change significantly (Number of VSMC/cross section in WT 181 ± 11 compared to 175 ± 18 in KO mice; mean ± SEM, n=3 for each genotype, p-value=0.7848 as determined by unpaired t-test. VSMC area 92.78 ± 3.30 in WT compared to 61.23 ± 2.28 in KO; mean ± SEM, n=3 for each genotype, p-value=0.0014 as determined by unpaired t-test, Number of VSMC/1000 µm² media in WT 6.9 ± 0.4 compared to 11.6 ± 0.6; mean ± SEM, n=3 for each genotype, p-value=0.0037 as determined by unpaired t-test). Quantitative measurements support our conclusion that the decreased medial wall thickness is due to decreased cell size but not due to decreased total cell number. (* indicates significance p<0.005, n=3/3 animals). The data clearly indicate the switch from contractile to synthetic VSMCs.
**Figure 11****: Changes in the morphology of vessels walls of mutant mice at different ages.** (A-B) Representative light microscopic images comparing the thickness of the media in WT and mutant vessel walls. Note the reduced cellular area of VSMCs and a decreased media thickness in mutant compared to WT mice. (C, D) Vessels of older mutant mice (18 month) did frequently contain neointimal lesions, which were never seen in WT mice.

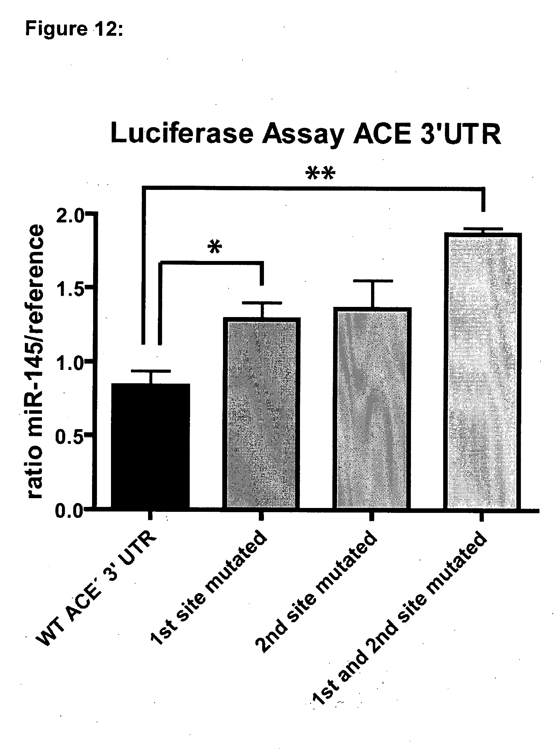

**Figure 12****: miR-145 specifically reduces activity of a luciferase-reporter under the control of the ACE 3' UTR.** Results of co-transfection experiments of a luciferase reporter plasmid carrying WT or mutated miR-145 bindings sites and a miR-145 expression vector in Hela-cells are shown. Higher luciferase activities, i.e. reduced repression by the co-transfected miR-145 plasmid was scored when reporter with mutations in the first or second miR-145 target or a combination of both were used. Changes were significant compared to the WT-vector (* p<0.05; ** p<0.001). A part of the ACE 3' UTR was PCR-amplified (GCTCGTGGCCACCGTGGGTCTCGCC, TAGGCCTTCCAAAGGATGGCTGA GG) and cloned into the Xbal site of pGL3 (Promega) immediately 3' of the luciferase ORF. Mutants of the UTR were constructed using mutated oligonucleotides and a two- or three step PCR. The first predicted miR-145 target site was mutated from GGAGTGTCCCATAAGAAACTGGA to GGAGTG-TCCCATAAGAAAtgaaA. The second target site was mutated from GGGAAGCCAGGGACAGGA to GGGAAGCCAGGGACAttc and a vector with mutation of both sites was prepared. 50% confluent Hela-1 cells on 24-well plates were co-transfected using Lipofectamine 2000 (Invitrogen) with luciferase vectors (80 ng) together with a renilla vector (pRL-TK, Promega; 20 ng) with 167 nM mmu-mir-145 miRIDIAN Mimic or a control-miR (Thermo Scientific Dharmacon), respectively. Each transfection was done in triplicate. Cells were lysed after 48 h and luciferase and renilla activities were determined using the Dual-Luciferase® Reporter Assay (Promega) and a Mithras LB940 plate reader (Berthold Technologies). miR-145 ratios were normalized to the respective controls.

The examples illustrate the invention.

### EXAMPLE 1: Experimental procedures

### Mice

A 129S7AB2.2 genomic BAC clone (bMQ-446B20; Sanger) was used to generate the targeting vector. Recombination in SW102 cell was employed to replace the miR-143 to miR-145 genomic region by a galK cassette (45) that was amplified by the primers flanking premiR-143 to premiR-145
(CACTCCTCCTGCCCAAGAAGAAAGCCGCGCGGCCTGCGTGCGGAGCGCCTGTCTCCCAGCggcgc gcctcagcactgtcctgctcctt;
TACTCCCCGGAAGGAAATGCCAGGGTGGGAGGGAGACAGATCCAGCTGCTAAGCCATGACggcgcg cctgttgacaattaatcatcggca). The genomic sequence was inserted via gap repair into a pKO targeting vector containing a DTA expression cassette and 450 bp homology arms for BAC recombination resulting in a vector containing 6.5 kb of sequence 5' of miR-143 and 5.5 kb 3' of miR-145 from genomic sequence TGAGCTGCTGGAGGCAAGGCTTGG to AGCCCAGCCTGGTCTATAGAGGGAG. The Ascl restriction sites flanking the galK cassette were used to replace the galK by a floxed IRESlacZ-NeoR cassette. The vector was electroporated into MPI II ES cells (46) and recombinant clones were identified. Recombinant ES cells were injected into blastocysts and transferred to pseudopregnant mice. Chimeric animals were backcrossed to C57BL/6 mice. Heterozygous animals were used to obtain WT and KO littermates. ES cells and mice were genotyped using an EcoRV restriction digest and a 5' outside probe amplified from genomic DNA using oligonucleotides (GACGGTCCTGAACACCTGGATCCC; TGCTGTGAGCTCTGAGAACCTCGG). For chronic ACE or AT1 inhibition drinking water of mice was supplemented with 600 µg/ml captopril (Sigma#C-8856) and 100 g/ml losartan (Fluka#61188) for at least 3 weeks. The experiments were performed in accordance with German legislation on protection of animals and were approved by the local governmental animal care committee (Regierungspraesidium Darmstadt).

### Morphological analysis

Arteries were perfused using a PBS solution via the heart apex at 100 mmHg pressure for 2 min, followed by perfusion with 4% paraformaldehyde in PBS. After the dissection of different arteries, these were additionally fixed in 3% glutaraldehyde for another 12 hours at 40°C and embedded in Epon. Ultrathin sections were stained with uranyl acetate and lead citrate, viewed and photographically recorded using a Philips CM 10 electron microscope. The phenotype of VSMC was determined ultrastructurally following criteria as previously reported (47). Morphological assessments were done by two different persons, who were unaware of the identity of analyzed samples, In brief, contractile VSMC are characterized by highly compacted contractile filaments and numerous intracellular electron dense puncta (arrowheads, Figure 3A), which strengthen and support the contractile apparatus, and prominent focal adhesions (arrows in Figure 3A). In contrast, synthetic VSMS are smaller in size and rich in ER and ribosomes compared to contractile VSMC and exhibit loosely packed contractile filaments, almost indiscernible focal adhesions, and intracellular electron dense puncta (Figure 3B). The mixed phenotype of VSMCs was defined by focal adhesions located at the cell periphery, scarceness or absence of intracellular electron dense puncta. In addition, ER structures in mixed VSMC are located only in the perinuclear region while they are extremely abundant and extent up to cell periphery in synthetic VSMC. These features clearly distinguish contractile VSMC from mixed VSMC. The phenotype of VSMC in each mouse was quantified in at least 300 cells per 3 arterial segments (minimum 100 µm) using consecutive transversal sections. For detection of -galactosidase activities tissues or embryos were fixed in PBS containing 1% formaldehyde, 0.2% glutaraldehyde, 0.2% NP-40 and 0.1% sodium deoxycholate for 15 - 60 min depending on size. Samples were processed as whole tissue or as cryosections. Samples were rinsed in PBS for 15 min and stained in PBS containing 2 mM MgCl2, 5 mM K3Fe(CN)6, 5 mM K4Fe(CN)6 1 mg/ml X-Gal and 0.2% NP-40 and 0.1% sodium deoxycholate. For embryos younger than E12.5 solutions contained 0.02% NP-40 and no sodium deoxycholate.

### Western blotting and Immunofluorescence.

10 g of protein lysates were separated on 4%-12% SDS-PAGE gradient gels and transferred onto nitrocellulose membranes (Invitrogen). Immunoreactive proteins were visualized with corresponding HRP-conjugated secondary antibodies using the SuperSignal West Femto detection solutions (Perbio Science). Signals were detected and analysed with a Biorad Versadoc System and Quantity One. We used polyclonal antisera against ACE-1 (Abcam#ab39172), NCS-1 (Santa Cruz#sc-13037), collagen type I (Sigma#C-2456) and monoclonal antibodies against "-smooth muscle actin (Sigma#C-6198), F4/80 (invitrogen#MF48004), caveolin-3 (BD#610420), caldesmon-1 (Sigma#C-4562; clone hHCD), GAPDH (NEB#2118) calponin (Sigma#C2687; clone hCP). Secondary antibodies were from Pierce: goat anti-rabbit HRP (#1858415), goat anti mouse HRP (#1858413) or Invitrogen (Alexa Fluor).

### Microarrays and Northern blot analyis

Tissues were dissected from PBS perfused animals and total RNA was isolated using the Trizol method (Invitrogen). RNA quality was checked on the Agilent 2100 Bioanalyser using the RNA 6000 Nano Kit. For mRNA expression analysis the Affymetrix GeneChip Mouse Genome 430 2.0 Array was employed with the respective one-cycle target labelling protocol. Data were analysed by the RMA algorithm using the Affymetrix Expression Console. Data are available at http://www.mpi-bn.mpg.de/en/research/heart/boettger/downloads.html An unpaired t-test was performed with log2-transformed data to identify significantly differentially expressed transcripts and a fold change was calculated using DNAStar ArrayStar3.0 software. 507 probe sets were identified with p<0.05 and FC>1.5 or FC<0.666. For miRNA expression analysis 384 3' C6 aminolinker DNA-oligonucleotides complementary to published miRNA (Sanger, miRBase) sequences were synthesised and spotted with eight replicates per array to Nexterion E slides. Probe labelling and hybridization followed the Agilent miRNA labelling protocol, but P-CTTTT-Alexa555 or P-CTTTTAlexa647 oligonucleotides (MWG-Biotech) were ligated to the miRNAs. Arrays were scanned and analysed with an Axon 4200B scanner and GenePix Pro 6.0. Normalisation and data analysis was performed using Acuity 4.0. For multiple tissue comparison, miRNA from tissues was labelled with the Alexa555 dye, miRNA from whole E15.5 embryos labelled with Alexa647 dye was used as a reference. Data were linear normalized to a log ratio of 1 for the ratio of medians of let-7a-g and let-7i signals. The log2 ratio data were centered for the single miRNA expression signals (by subtracting the row mean from each value), log2 ratio data are visualized. For Northern blot analysis 3 µg (aorta 1.5 µg) of total RNA were separated on a 15% Polyacrylamide-Urea gel (Invitrogen) and blotted on Hybond XL (Amersham). miR-145, miR-143 and U6 antisense oligonucleotides were labeled with γ-ATP and PNK (NEB) and purified using Micro Bio-Spin® 6 columns (Biorad). Hybridization was performed in Ultrahyb hybridization buffer (Ambion) at 30°C over night. Blots were washed 2 times in 2 x SSC/0.1 % SDS at 25°C (5 min) and at 42°C (15 min). Signals were detected using a BAS-2500 imager (Fujifilm).

### Quantitative proteomics

Aortae were dissected from 13C6-lysine labelled "heavy" WT mice and from non-labeled wildtype and knockout litter mates. Protein extracts were processed and mass spectroscopy was performed as described (15). Briefly, all LC-MS/MS measurements were performed with an LTQ-Orbitrap (Thermo Fisher Scientific) combined with an Agilent 1200 nanoflow HPLC system. The mass spectrometer was operated in the data-dependent mode to automatically measure full MS scans and MS/MS spectra. Peptides were identified by searching against the International Protein Index sequence database (mouse IPI, version 3.24) using the Mascot search algorithm (www.matrixscience.com). Mass spectra were analyzed by the MaxQuant software package (48, 49), which performs peak lists, SILAC quantification and false positive rate determination (50). Ratio and p-values were calculated from the mean of three 13C6Lys- WT/WT and three 13C6Lys-WT/KO ratios.

### Identification of miRNA targets

miRNA target predictions were downloaded from miRBase (http://microrna.sanger.ac.uk; Mus musculus Targets Release Version v5, release: 2008-01-08, miRanda-based), from microrna.org (http://www.microrna.org/; mouse miRNA Target Site predictions release 2008-01-08; miRanda-based) (17) and from TargetScan.org (www.targetscan.org, v4.2, predicted targets of conserved families) (19).

Lists of significantly regulated mRNAs WT vs KO, but not affected by chronic captopril treatment (via Affymetrix ID) or proteins (via gene symbol) were compared to lists of targets predicted for miR-143 and miR-145 using the MatchMiner database at http://discover.nci.nih.gov/matchminer/ (20).

### Hemodynamic measurements

Aortic catheterization was performed using a 1.4F pressure-volume catheter. Data were recorded and analyzed with Chart v5.4 as described before (51). Mice were anaesthetized with isoflurane (2 vol% in 02) and their body temperature was kept at 37°C as monitored by a rectal probe. Captopril (30 mg/kg body weight, 50 µl) and angiotensin II (5 to 50000 ng/kg body weight, 50 µl) were applied via the left jugular vein. For measurements of conscious, unrestrained mice, hemodynamic parameters were recorded by telemetry (DSI, Transoma Medical, USA, TA11PA-C10 transmitters) after a recovery period from surgery of more than 7 days. Recordings were obtained every 2 minutes for 20 seconds at a sampling frequency of 1000 Hz and were analyzed for day (07:00 - 19:00) and nighttime (19:00 - 07:00) periods, respectively, to evaluate circadian variation in hemodynamics.

### Myograph experiments

Isolated segments of femoral and mesenteric arteries were examined in an isometric microvessel myograph as described previously (52). Mounted vessels were stimulated with 80 mM K+, 30 nM angiotensin II or increasing concentrations of phenylephrine (100 nM to 10 µM). The pCa-force relationships (13, 14) were obtained in permeabilized mouse femoral artery segments. In brief, arteries were permeabilized for 30 minutes using 0.5 % Triton X-100 in relaxation solution. The latter consisted of (in mM): EGTA 1, Pipes 30, Na2ATP 5.16, MgCl2 7.31, sodium creatine phosphate 10 and potassium gluconate 74.1. For contraction curves the buffer was supplemented with 160 U/ml calmodulin and 100 U/ml creatine phosphokinase. The concentration of free Ca2+ ions was calculated as described previously (53).

### Analysis of blood gas and gut transit time

Blood was collected after decapitation of mice and was analysed using an i-Stat 1 Analyser with EG6+ cartridges. For analysis of gut transit time, after a 20 h fasting period mice were force fed with 0.3 ml 2.5 % Evans Blue (Sigma#E-2129) in 1.5 % methylcellulose (Fluka#64632). Mice were returned to cages with water and food and time until appearance of stained faeces was recorded.

### Analysis of blood circulating angiotensin II and aortic cGMP concentration

Blood serum samples were obtained by cardiac puncture using Bestatin angiotensinase inhibitor solution (Bühlmann GmbH, Germany). Angiotensin II concentrations were determined using a angiotensin II RIA after reverse phase extraction of plasma samples using columns provided with the RIA (Bühlmann GmbH, Germany). To match the volume requirement of the RIA a part of the plasma samples analysed (n=5/4; WT/KO) were obtained by pooling plasma of max. two animals of the same genotype. Aortae from WT and KO mice were snap frozen in liquid nitrogen and homogenized in 5 % TCA. cGMP concentration was determined with a cGMP EIA Kit (Cayman Chemicals) using the acetylated cGMP protocol.

### Data analyses and statistics

Data are presented as means ± SEM and tested by t test, one or two way ANOVA. P values less than 0.05 were considered to be statistically significant.

### EXAMPLE 2: Expression of miR-143/145 and generation of miR-143/145 mutant mice

To understand the role of miRNAs in the development and regulation of the cardiovascular system a series of miRNA microarray hybridization experiments using a number of different mouse tissues derived from various developmental stages was performed. This microarray analysis revealed that the expression of miR-143 and miR-145, which form a small cluster on mouse chromosome 18, strongly correlated with the number of smooth muscle cells in all organs studied (Figure 1A). Detailed analysis of the function of miRNAs is often hampered by the lack of a robust expression map at the single-cell level. Therefore the miR-143/145 coding genomic region was replaced with a lacZ reporter by fusing the premiR-143 sequence to an IRES-LacZ sequence. This manipulation deleted the sequences coding for the mature miR9 143 and miR-145 sequences and a 1.3 kb fragment located between both genes (Figure 1 B, C) and allowed us to monitor transcription of the miR-143 gene. Examination of the activity of the lacZ-reporter in heterozygous mice revealed that the miR-143 locus was active during early stages of heart development from E8.5 onwards (E9.5, Figure 2A) but disappeared from cardiomyocytes at later stages (E16.5; Figure 2B) and became exclusively confined to smooth muscle cells of the cardiovascular system, the gastrointestinal tract, the bladder, uterus and lung (Figure 2C-F). Interestingly, other smooth muscle genes such as smooth muscle α-actin and SM22α follow a similar path and are also found in embryonic cardiomyocytes at early stages of heart development (10, 11). In the esophagus an initial expression of the reporter was observed at P0 in the muscularis mucosae and the muscularis externa, which are composed of smooth muscle cells at this stage. In adult mice expression of miR-143/145 was only found in the muscularis mucosae since skeletal muscle cells supersede smooth muscle cells in the muscularis externa of the esophagus during postnatal development (Figure 8) (12). The postnatal expression of the miR-143/145 cluster in VSMCs was relatively stable and did not respond to trans-stenotic pressure gradients induced by transverse aortic constriction (n=3/3; measured by microarray hybridization, data not shown).

### EXAMPLE 3: Loss of miR-143/145 leads to a shift of contractile to synthetic VSMCs

To study consequences of the loss of miR-143/145 heterozygous miR-143/145 knockout animals were crossed. Homozygous mutants were viable and fertile and did not show gross macroscopical alterations. Microarray-based and northern blot-based miRNA expression analysis of different knockout tissues confirmed the loss of the expression of both miRNAs and demonstrated the specificity of the signals detected by microarray hybridization (Figure 1D, E). To obtain a more detailed view about the function of the miR-143/145 gene cluster a comprehensive analysis of the structure and function of VSMCs *in vivo* was performed. Major structural changes of arterial VSMCs were noted in all arteries investigated (Figure 3). Electron microscopy analysis revealed a severe reduction of the number of contractile VSCMs and an increase of synthetic VSMCs in the aorta and the femoral artery of knockout animals (Figure 3A-C, H-J), which went along with a reduced thickness of the smooth muscle layer of the femoral artery (WT 32.9±1.4 vs. KO 19.5±0.6 µm, n=8/7, p<0.0001, Figure 3HJ). The phenotype switch of smooth muscle cells from contractile to synthetic was established by both analysis of smooth muscle shape and number of dense bodies per VSMC area (Figure 10). Morphometric measurements indicated that the reduced size of the muscle layer was due to a smaller size of VSMCs (Figures 10, 11) and not to a reduced total number of VSMCs (Figure 3H,I and Figures 10, 11). The aorta showed a less severe phenotype compared to the femoral artery (not shown). No differences in the number of proliferating or apoptotic smooth muscle cells was apparent in arteries of adult mutants (not shown). The invariant total number combined with the reduction of the size of VSMCs, did reflect the shift between contractile and synthetic phenotypes and suggested that the loss of miR-143/145 does not affect differentiation of VSMCs *per se* but the ability to adjust the phenotype to the physiologically required contractile state.

### EXAMPLE 4: The miR-143/145 cluster is required for normal contractility of arteries in vitro and regular blood pressure in vivo

Next the contractility of arterial smooth muscle in isolated arterial rings of WT and KO mice was analyzed directly. Reduced contractility of femoral artery explants from KO mice (-39.1%, down from 4608+596 µN to 2793+214 µN, n=8-12 vessels per genotype, p<0.05) was observed after stimulation with 80 µM extracellular potassium, which causes cellular depolarization and therefore probes the basic ability for contraction (Figure 4A). To analyze whether KO mice show specific deficits to contractile cues that might act on top of a more unspecific impairment of contraction the response of artery explants to the two most important regulatory pathways of the vascular tone, adrenergic and angiotensin signalling was tested. A nearly complete loss of the ability of artery explants to contract in response to angiotensin II stimulation and a dramatic deficit after stimulation with the α1-adrenoceptor agonist phenylephrine was observed (Figure 4B-D). Another questions was whether the contractile deficit of KO vessels was due to lower Ca2+ sensitivity of the myofilaments and/or due to altered receptor signalling. Therefore femoral artery segments with Triton X-100 were permeabilized and pCa2+-force relationships were measured (Figure 4F) (13, 14). Maximal Ca2+-induced vasoconstriction was significantly blunted in KO vs. WT vessels (-31%, WT: 1228+92 µN, KO: 846+88 µN, n=8-12 vessels per genotype, p<0.05). Furthermore, the pCa2+ curves were shifted leftwards in KO vs. WT femoral arteries (pEC50 KO: 6.113 ± 0.005, WT 5.785 ± 0.007, p<0.001) indicating that Ca2+ sensitivity was slightly increased in KO vessels. In principle, the contractile deficit of KO vessels might be caused by the accumulation of synthetic smooth muscle cells, which are unable to develop the same force as contractile smooth muscle cells, by a general defect in all smooth muscle cells, or a combination of both. To distinguish between these possibilities maximal vasoconstrictory effects to the number of contractile cells in the media were normalized. Interestingly, normalized contractile force elicited by Ca2+ in skinned vessels or by K+ depolarization in intact vessels, did not differ between WT and miR-143/145 mutant mice (n=8-12 vessels per genotype, p<0.05). However, normalized receptor-mediated responses induced by angiotensin II and phenylephrine were severely reduced (95% for angiotensin II and 85% for phenylephrine) in KO vessels (n=8-12 vessels per genotype, p<0.01 for angiotensin II and p<0.001 for phenylephrine) suggesting that the lack of miR-143/145 impairs receptor-mediated responses in contractile smooth muscle cells. Of course, defects in receptor-mediated contraction of remaining contractile VSMCs is almost certainly only one reason for the impaired contractility of vessels in miR-143/145 knockout mice. The presence of synthetic smooth muscle cells, which show a major contractile defect, in mutant vessels will also contribute to the contractile deficit of KO vessels. Taken together it was demonstrated that multiple mechanisms, which include accumulation of synthetic VSMCs and the inability of remaining contractile VSMCs to contract in response to receptor-mediated signals, contributed to reduced contractility of artery explants. It is reasoned that the morphological alterations of VSMCs and decreased contractility of arteries might also affect the vascular tone of arteries in mutant animals *in vivo.* Intravascular blood pressure measurements disclosed reduced systolic and diastolic blood pressure in mutant compared to control animals under anaesthesia (systolic pressure WT 100±5.5 mm Hg vs. KO 86±2.4 mm Hg, n=6/6, p<0.05; diastolic WT 70±7.0 mm Hg vs. KO 49±4.4 mm Hg, n=6/6, p<0.05). To avoid potential artifacts caused by anaesthesia we also measured blood pressure telemetrically using catheters implanted into the femoral artery. Again, systolic blood pressure was significantly reduced in KO animals while the heart rate was not altered (Figure 5A,B). Intravenous application of angiotensin II resulted in a compromised increase of blood pressure in knockout compared to WT mice (Figure 5C). To test whether the reduced pressure response was due to desensitization of VSMCs by increased angiotensin II signalling *in vivo,* the formation of angiotensin II by administration of captopril was blocked. Acute inhibition of ACE led to a pronounced reduction of diastolic pressure in KO animals, which corresponded to the increased angiotensin II signalling (Figure 5D). A reduced cardiac ventricle/body weight (WT 4.93±0.09 mg/g vs. KO 4.44±0.12 mg/g, n=12/14, p<0.01) and reduced ventricle weight/tibia length ratios (WT 8.47±0.24 mg/mm vs. KO 7.67±0.27 mg/mm, n=12/14, p<0.05) probably as a result of the reduced blood pressure while body weights did not differ between miR-143/145 mutant and wild-type mice was also detected. To rule out a potential contribution of other smooth muscle cell containing organs (lung, gastrointestinal tract) to the hypotensive phenotype a number of additional physiological parameters were examined. (i) Analysis of blood gas composition did not uncover changes in pO2, pCO2, BEecf, HCO3, TCO3, sO2 (n=5/5, data not shown) indicating that the pulmonary gas exchange was not compromised in KO mice. (ii) No signs of cardiac malfunctions or right ventricular hypertrophy secondary to changes in pulmonary function (mean right ventricular myocyte cross-sectional area: WT 214.1±14.4 µm2 vs. KO 221.2±17.5 µm2, n=6/7) were noted. (iii) Transit time through the gastrointestinal tract was unaltered (WT 250±21 min vs. KO 260±16 min, n=6/6).

### EXAMPLE 5: miR-143 and miR-145 modulate regulators of smooth muscle cells at different levels

To elucidate the molecular changes, which resulted in the impairment of the contractile phenotype in VSMCs of miR-143/145 knockout animals a novel high-throughput approach was utilized. This experimental system was based on quantitative, mass spectrometry (MS)-based proteomics using reference mice that were completely labeled with 13C6Lys-substituted version of lysine ("SILAC mice") (15), combined with microarray-based transcriptional profiling. SILAC analysis from aortae lacking expression of miR-143/145 (n=3/3) was employed to determine the relative changes of approximately 1600 proteins in mutant mice relative to wildtype controls (Figure 6A-E, Table 2,3). The SILAC approach was corroborated by Western Blot analysis and immunofluorescence staining of selected proteins (Figure 6H, Figure 9). In addition, mRNA expression profiles of aortae from knockout and wildtype animals using Affymetrix GeneChips (n=5/5) was analyzed to identify possible similarities and differences between changes in protein and mRNA levels in knockout and wild-type animals (Table 4, 5). Since miRNAs do not only affect the stability of mRNAs but also the efficiency of protein translation this approach allowed us not only to identify the maximum number of potentially affected proteins but also to gain an idea about the mechanism employed. Transcripts and proteins, which differed significantly between WT and knockout tissues were matched to potential target transcripts predicted by the miRanda (16) (microRNA.org) database (17) and miRBase (18)) or TargetScan 4.2 algorithm (19) for miR-143 and miR-145 using the MatchMiner tool (20). For the analysis those transcripts were omitted, which were normalized by extended treatment with the ACE-inhibitor captopril or the AT1-receptor inhibitor losartan (see below) since they are unlikely to represent primary targets of miR-143/145. An up-regulation of miR-143/145 mRNA targets was found, which are known to play important roles in the biology of smooth muscle cells (miR-145 target: Argagp12; Tpm4 (tropomyosin 4) is target of both miR-143 and miR-145) (1). In addition, several other mRNAs were increased, which fulfil tasks in various other cell types but lack an apparent specific function in VSMCs. The unbiased protein analysis did reveal the rise of predicted targets of miR-143/145 such as Tpm4, which were also increased at the mRNA level. As expected, the up-regulation of putative target proteins that were not met by transcriptional changes was also detected. Most likely these proteins were controlled by miR-143/145-mediated translational repression. Interestingly, one of the most strongly affected proteins in this group was ACE, which converts circulating angiotensin I into its active form angiotensin II (Figure 6J). The 3'UTR and the open reading frame of mouse ACE contain several miR-145 binding sites with high scores and low energy as predicted by the miRanda algorithm (16) (Figure 51). To test the functional significance of the putative miR-145 target sites in the 3'UTR we employed a luciferase reporter assay. It was found that intact but not mutant miR-145 target sites are able to mediate repression of reporter gene activity upon overexpression of miR-145 (Figure 12). The miRanda algorithm also detected miR-145 binding sites in the UTR and ORF of ACE mRNA of other species such as chick, rat and humans. Angiotensin II is not only a potent agonist for the contraction of VSMCs but also a major regulator of the contractile phenotype of VSMCs, which explains - at least in part - the shift from the contractile to the synthetic phenotype in miR-143/145 knockout mice. Most likely this mechanism synergizes with effects of miR-143/145 on other targets that are up-regulated in knockout mice. In line with this premise additional changes in the transcript and/or protein expression level of molecules known to influence the SMC phenotype were observed (Table 2-5). TPM-4, which is a predicted target of both miR-143 and miR-145, is a structural protein that is specifically up-regulated in synthetic smooth muscle cells (21). The up-regulation of ACE in VSMCs of mutant mice raised the question whether the local rise of ACE concentrations does also suffice to increase systemic angiotensin II levels. Interestingly, the level of blood circulating angiotensin II was not up-regulated in KO vs. WT (KO: 16+3, WT 28+6 fmol/ml plasma (n=9 KO / 8 WT animals, pooled to n=4/5 plasma samples), which might explain phenotypic differences of miR-143/145 mutant mice compared to other transgenic models that rely on the unrestricted overexpression of components of the RAAS. In addition to the up-regulation of predicted targets, changes in the expression of numerous mRNAs and proteins were observed, which occurred most likely due to secondary events. Among others a down-regulation of angiotensin receptor 1 (*Agtr1b*) was detected, which is a typical response to excessive angiotensin II signalling. The down-regulation of angiotensin receptor 1 corresponded to reduced cGMP-concentrations in the aortae of KO compared to WT mice (KO: 30.0+6.7; WT: 51.1+7.9 pmol cGMP/g wet weight, n=3/3). Angll is known to stimulate cGMP production, an effect, which was apparently blocked, due the down-regulation of angiotensin AT1 receptor. A reduced expression of Agtr1-interacting molecules Freq/NCS-1 (Western Blot, Figure 9 / RNA, Table 5) / Kcnd3-KV4.3 (RNA) (22) and of molecules that control the trafficking of plasma membrane receptors such as Cav-2 (SILAC, Table 3) and Cav-3 (WB, Figure 9) was also found. In contrast, cholecystokinin, and gastrin releasing peptide (Table 4), which are involved in the regulation of contractility of smooth muscle cells, were up-regulated. In principle, many elements of signalling cascades, which govern contraction and migration of smooth muscle cells were changed including a down-regulation of RGS4, RGS5, RGS7bp (Table 5) and up-regulation of the RGSinteracting molecule GNB5 as well as of components of the Rho signalling cascade (Rock1, Rnd2/3, Cdc42ep3, Arggef17) and molecules that direct calcium handling and signalling (SERCA, Caldesmon-1, Camk2g) of VSMCs. The effects on the Rho signalling cascade might be of particular significance since it affects nuclear translocation and/or activation of SRF (23). In addition, several molecules (CPI-17, MYLK-Telokin, MYPT1; Table 3, 5) were repressed which directly regulate contraction of VSMCs by controlling phosphorylation of myosin light chain. Changes in the expression levels of α-SM actin and SM MyHC according to our in vivo SILAC measurements were not detected. Interestingly however, a loss of the expression of several other markers of differentiated smooth muscle cells and an increase of the expression of skeletal muscle markers was noted (Table 3, 4).

### EXAMPLE 6: ACE inhibition partially rescues loss of miR-143/145

To directly test the relevance of the proposed miR-145 target ACE for the observed phenotype ACE and the AT1 receptor was chronically inhibited, by oral administration of captopril and losartan, respectively. According to our model the lack of ACE repression in miR-143/145 knockout animals causes a local increase of angiotensin II signaling in smooth muscle cells, which should be rescued by pharmacological inhibition of ACE. It was found that a 20 day treatment with captopril markedly improved the contractile response of mutant, but not of WT arteries to phenylephrine and angiotensin II (Figure 4B,D). Maximal angiotensin II-induced force of contraction was 3.2 ± 0.7 mN in untreated WT vessels as compared with 3.4 ± 0.6 mN in captopril-treated WT vessels (p=0.84, 12/8 untreated/treated WT vessels). In femoral arteries from KO mice, contractile force was greater after captopril-treatment (0.30 ± 0.05 mN) than in untreated KO specimens (0.11 ± 0.03 mN, p=0.007, 9/9 treated/untreated KO vessels). While the absolute functional improvement after ACE inhibition was significant but relatively small for angiotensin II-induced contraction (9% of K+ contraction, Fig. 4B) phenylephrine-effects increased to 46% of the K+ contraction (Fig. 4D) after ACE inhibition. In contrast, the maximal force of contraction of KO arteries generated by K+- induced depolarization did not improve (Figure 4A). Similarily, treatment of KO mice with the AT1 receptor antagonist losartan for 3 weeks resulted in an enhancement of vasoconstriction of phenylephrine induced contraction by 214% or 57% at 3 µM and 10 µM phenylephrine, respectively, which corresponds to 96% of the K+ force (Figure 4E). In WT mice, losartan did not affect phenylephrine-induced vasoconstriction (Figure 4E). Microarray analysis revealed that the partial rescue of agonist-induced contractility was accompanied by normalization of several transcripts such as Rock1, SERCA, caldesmon-1 and Camk2g (Table 1 which are involved in receptor-mediated contractility of smooth muscle cells and calcium signaling. In total, 248 out of the 507 probe sets, which were deregulated in mutant compared to WT mice (p<0.05, FC>1.5 or FC<0.666, n=5/5), returned to normal levels after 3 weeks of oral application of captopril to mutant mice (WT/KOcaptopril, n=3) (Table 1). Similarly, a normalization of 220 out of the 507 probe sets (p<0.05; FC>1.5 or FC<0.666; n=5/5) after 3 weeks of oral application of the AT1 receptor blocker losartan to ,mutant mice was observed (WT/KOiosartan, n=3), (p>0.05, n=3), (Table 1). It was also found that the probe sets, which were normalized by either captoril or losartan, strongly overlapped. 164 out of the 248 and 220 probe sets were identical. Although this experiment clearly proves the functional relevance of the miR-145 target ACE for the contractile dysfunction, significant morphological changes in arteries of KO mice after 20 days treatment with captopril or losartan were not observed (data not shown).

### EXAMPLE 7: Development of neointimal lesions

Transitions of the phenotypic state of VSMCs play a key role in the repair of vascular injuries and in the development and/or progression of atherosclerosis, which is also reflected by the rapid down-regulation of multiple miRNAs during neointima development after vascular injury (24). Therefore, the focus was turned to long-term changes of the vascular system that might arise as a result of the accumulation of synthetic VSMCs. Importantly, neointimal lesions in the femoral arteries of 18 months old miR-143/145 mutant mice (n=7) were found, which comprised 49.12+5.75% of the media. Neointimal lesions were absent in wild-type controls (n=9) and negligible in younger miR-143/145 mutants (n=8), (Figure 11). Early lesions in miR-143/145 mutant mice were characterized by the presence of smooth muscle cells in the neointima (Figure 7 B, E, G) while more mature lesions contained large amounts of smooth muscle cells and macrophages and deposits of amorphous collagen I, which resulted in the formation of large plaques (Figure 7 C, F, H, K, M, N). Lipid rich cores or foam cells within the lesions, which distinguishes them from classic atherosclerotic plaques were never observed. Similarly, the localization of plaques in the femoral arteries and not the aortic arch discriminates miR-143/145 knockout mice from other disease models, which are based on the massive increase of serum lipoproteins such as in apoE-deficient mice (25). In line with these findings normal serum concentrations of triglycerides and lipoproteins in miR-143/145 knockout mice were found (data not shown).

### TABLES:

**Table 1: Treatment of mutant mice with the ACE inhibitor captopril or the AT1 inhibitor losartan normalizes expression of numerous dysregulated transcripts.**

| Affymetrix | Gene | miR-143/145 KO | | miR-143/145 KO | | miR-143/145 KO | |
|---|---|---|---|---|---|---|---|
| Probe Set ID | Symbol | vs. WT FC | p-value | captopril FC vs. WT p-value | | losartan FC vs. WT p-value | |
| 1417464_at | Tnnc2 | 5,57 | 0,028 | 1,13 | 0,699 | 1,51 | 0,309 |
| 1427735_a_at | Acta1 | 4,74 | 0,023 | 0,87 | 0,711 | 0,76 | 0,289 |
| 1448756_at | S100a9 | 3,48 | 0,038 | 0,78 | 0,643 | 1,64 | 0,246 |
| 1419312_at | Serca1 | 3,36 | 0,017 | 0,72 | 0,145 | 0,82 | 0,294 |
| 1419394_s_at | S100a8 | 3,16 | 0,042 | 0,81 | 0,636 | 1,83 | 0,108 |
| 1448371_at | Mylpf | 2,75 | 0,027 | 0,77 | 0,223 | 1,05 | 0,759 |
| 1422926_at | Mc2r | 2,68 | 0,001 | 1,23 | 0,370 | 0,70 | 0,198 |
| 1450468_at | Myoc | 2,13 | 0,001 | 0,99 | 0,940 | 0,76 | 0,309 |
| 1419871_at | Ppp2r1b | 1,90 | 0,030 | 0,81 | 0,331 | 0,65 | 0,080 |
| 1420647_a_at | Krt8 | 1,78 | 0,022 | 1,09 | 0,686 | 1,00 | 0,992 |
| 1433486_at | Clcn3 | 1,73 | 0,003 | 1,09 | 0,474 | 1,27 | 0,085 |
| 1427183_at | Efemp1 | 1,71 | 0,005 | 1,22 | 0,379 | 0,71 | 0,182 |
| 1433694_at | Pde3b | 1,71 | 0,037 | 1,04 | 0,894 | 0,87 | 0,548 |
| 1435184_at | Npr3 | 1,61 | 0,017 | 1,00 | 0,996 | 1,68 | 0,035 |
| 1455158_at | Itga3 | 0,48 | 0,002 | 0,76 | 0,053 | 0,84 | 0,259 |
| 1440789_at | Neo1 | 0,49 | 0,002 | 1,07 | 0,654 | 1,27 | 0,084 |
| 1423222_at | Cap2 | 0,52 | 0,003 | 0,77 | 0,151 | 1,36 | 0,072 |
| 1449522_at | Unc5c | 0,41 | 0,005 | 0,63 | 0,112 | 0,80 | 0,281 |
| 1424768_at | Cald1 | 0,50 | 0,008 | 1,09 | 0,515 | 1,53 | 0,002 |
| 1460729_at | Rock1 | 0,45 | 0,009 | 0,99 | 0,944 | 1,77 | 0,050 |
| 1423942_a_at | Camk2g | 0,58 | 0,011 | 0,87 | 0,362 | 1,17 | 0,490 |
| 1433682_at | Arhgef17 | 0,63 | 0,016 | 0,82 | 0,210 | 1,11 | 0,476 |
| 1449876_at | Prkg1 | 0,45 | 0,017 | 1,12 | 0,671 | 1,16 | 0,545 |
| 1425506_at | Mylk | 0,70 | 0,026 | 0,90 | 0,202 | 1,06 | 0,598 |

Affymetrix GeneChip® analysis revealed 507 probe sets that were significantly changed between WT and KO (p<0.05; FC>1.5 or FC <0.666; n=5/5). 248 of these probe-sets showed WT levels after chronic ACE inhibition for 20 days (p>0.05, n=3). 220 of the 507 probe sets were not significantly changed after 20 days of losartan (p>0.05, n=3) treatment. Losartan and captopril both suppress changes induced by up-regulated expression of the miR-145 target ACE. Therefore the effects of both inhibitors strongly overlap: expression of 164 probe sets was normalized by both losartan and captopril.

**Table 2: SILAC-detected proteins up-regulated in aortae miR-143/145 KO vs. WT. A selection of proteins is shown that are detected to be significantly up-regulated in the SILAC experiment (p<0.05, n=3/3).**

| **Protein Names** | **Symbol** | **ratio** |
|---|---|---|
| Quinone oxidoreductase; Crystallin, zeta | Cryz | 9.2 |
| protein N-myristoyltransferase 1 | Nmt1 | 6.7 |
| CD166 antigen precursor | Alcam | 5.5 |
| collectin sub-family member 12 | Colec12 | **5.1** |
| **Angiotensin-converting enzyme** | **Ace** | **4.9** |
| Thiosulfate sulfurtransferase | Tst | **4.2** |
| Malic enzyme 1 | Me1, Mod-1, Mod1 | 3.8 |
| Adenosine kinase | Adk | **3.6** |
| myosin heavy chain 10, non-muscle | Myh10 | **3.6** |
| FK506-binding protein 3 | Fkbp3, Fkbp25 | **3.4** |
| Ectonucleotide pyrophosphatase | Enpp1, Npps | **3.4** |
| Tubulin beta-4 chain | Tubb4 | **3.3** |
| Tubulin beta-5 chain | Tubb5 | **3.1** |
| Fatty acid-binding protein, heart | Fabp3, Fabph1 | **3.1** |
| Versican core protein precursor | Vcan, Cspg2 | **2.7** |
| Glia maturation factor beta | Gmfb | **2.7** |
| Protein disulfide-isomerase A3 precursor | Pdia3, Erp | **2.7** |
| Hyaluronan and proteoglycan link protein 1 precursor | Hapln1, Crtl1 | **2.7** |
| Heat shock protein 90 kDa beta member 1 | Hsp90b1, Tra-1 | **2.6** |
| Epoxide hydrolase 2 | Ephx2, Eph2 | **2.6** |
| Stress-associated endoplasmic reticulum protein 2 | Serp2, Ramp4 | **2.5** |
| Ubiquitin carboxyl-terminal hydrolase 5 | Usp5, Isot | **2.4** |
| Leukocyte elastase inhibitor A | Serpinb1 a | **2.3** |
| **Tropomyosin alpha-4 chain;Tropomyosin-4** | **Tpm4** | **2.1** |
| Glutathione S-transferase | Gstm1, Gstm3 | **2.1** |
| 4F2 cell-surface antigen heavy chain | Slc3a2, Mdu1 | **2.1** |
| Platelet-activating factor acetylhydrolase IB subunit beta | Pafah1b2 | **2.0** |
| Insulin degrading enzyme | Ide | **2.0** |
| Microtubule-associated protein 1B | Map1b, Mtap5 | **2.0** |
| Golgi integral membrane protein 4 | Golim4 | 1.9 |
| Enolase 3 | Eno3 | 1.8 |
| Hook homolog 3 | Hook3 | 1.7 |
| Nucleoside diphosphate kinase A | Nme1 | 1.7 |
| CD31 antigen | Pecam1 | 1.7 |
| Fibrinogen gamma chain precursor | Fgg | 1.7 |
| Spectrin alpha 2 | Spna2 | 1.7 |
| myosin regulatory light chain A, smooth muscle homolog; | 2900073G15Rik | 1.6 |
| Elongation factor 2;EF-2 | Eef2 | 1.6 |
| Calnexin precursor | Canx | 1.6 |
| Aldose reductase homolog | 2310005E10Rik | 1.6 |
| Glutathione S-transferase Mu 5 | Gstm5 | 1.6 |

**Table 3: SILAC-detected proteins down-regulated in aortae miR-143/145 KO vs. WT. A selection of proteins is shown, which was significantly down-regulated in the SILAC experiment (p<0.05, n=3/3). Selected markers of smooth muscle contraction are marked with an *.**

| **Protein Names** | **Symbol** | **ratio** |
|---|---|---|
| ribosomal protein L7 | Rpl7 | **0.1** |
| Calcium/calmodulin-dependent protein kinase type II gamma chain | Camk2g* | **0.1** |
| Tubulin polymerization-promoting protein family member 3 | Tppp3 | **0.2** |
| Eukaryotic translation initiation factor 2C 2;Argonaute-2;S | Eif2c2, Ago2 | **0.3** |
| Melanoma-associated antigen MUC18;CD146 antigen | Mcam | **0.4** |
| Nuclear mitotic apparatus protein 1 homolog | Numa1 | **0.4** |
| Serine/threonine-protein phosphatase PP1-beta catalytic subunit | Ppp1cb | **0.4** |
| protein kinase c delta-bindig protein homolog | Prkcdbp | **0.4** |
| Laminin subunit beta-2 | Lamb2 | **0.4** |
| Cytoglobin | Cygb | **0.4** |
| Smoothelin | Smtn * | **0.4** |
| Laminin subunit gamma-1 precursor;Laminin B2 chain | Lamc1, Lamb-2 | **0.4** |
| Histone H2A type 2-A | Hist2h2aa1 | **0.4** |
| Glutamyl aminopeptidase;CD249 antigen | Enpep | **0.5** |
| Histone H4 | Hist1h4a | **0.5** |
| Insulin-like growth factor-binding protein 7 | Igfbp7, Mac25 | **0.5** |
| Laminin subunit alpha-4 precursor | Lama4 | **0.5** |
| CPI-17, Protein phosphatase 1 regulatory subunit 14A;17 kDa | Ppp1 r14a, Cpi17* | **0.5** |
| Heterogeneous nuclear ribonucleoprotein D-like | Hnrpdl | **0.5** |
| Voltage-dependent calcium channel subunit alpha-2/delta-1 | Cacna2d1 | **0.5** |
| Histone H2B type 1-H | Hist1h2bh | 0.6 |
| Calcium-binding protein 39 | Cab39 | 0.6 |
| MLCK Myosin light chain kinase, smooth muscle | Mylk* | 0.6 |
| Tenascin X;Tenascin-X | Tnxb | 0.6 |
| CD81 antigen | Cd81 | 0.6 |
| Clusterin precursor | Clu, Apoj | 0.6 |
| Integrin alpha-5 precursor | Itga5 | 0.6 |
| Sodium/potassium-transporting ATPase subunit beta-3 | Atp1b3 | 0.6 |
| PDGF-associated protein | Pdap1 | 0.6 |
| UNR-interacting protein | Strap, Unrip | 0.6 |
| NADPH-dependent carbonyl reductase | Dhrs4, D14Ucla2 | 0.6 |
| Glycolipid transfer protein;GLTP | Gltp | 0.6 |
| Caveolin-2 | Cav2 | 0.6 |

**Table 4: Selected transcripts detected up-regulated in miR-143/145 KO vs. WT aortae. A selection of transcripts, which was up-regulated in the Affymetrix GeneChip analysis, is shown (p<0.05, n=5/5; see methods). Selected signaling molecules, markers of smooth muscle contraction and of skeletal muscle differentiation are marked with #,* and §, respectively.**

| **Probe Set ID** | **Gene Title** | **Gene Symbol** | **FC** |
|---|---|---|---|
| 1417464_at | troponin C2, fast | Tnnc2 § | 5.57 |
| 1435354_at | potassium inwardly-rectifying channel, subfam. J, member 15 | Kcnj15 | 5.13 |
| 1427735_a_at | actin, alpha 1, skeletal muscle | Acta1 § | 4.74 |
| 1419608_a_at | melanoma inhibitory activity 1 | Mia1 | 4.68 |
| 1416889_at | troponin I, skeletal, fast 2 | Tnni2 § | 4.24 |
| 1425425_a_at | Wnt inhibitory factor 1 | Wif1 | 3.84 |
| 1448756_at | S100 calcium binding protein A9 (calgranulin B) | S100a9 | 3.48 |
| 1419312_at | ATPase, Ca++ transporting, cardiac muscle, fast twitch 1 | Atp2a1/SERCA | 3.36 |
| 1434411_at | collagen, type XII, alpha 1 | Col12a1 | 3.27 |
| 1419473_a_at | Cholecystokinin | Cck # | 3.17 |
| 1419394_s_at | S100 calcium binding protein A8(calgranulin A) | S100a8 | 3.16 |
| 1418480_at | pro-platelet basic protein | Ppbp | 2.82 |
| 1448371_at | myosin light chain, phosphorylatable, fast skeletal muscle | Mylpf § | 2.75 |
| 1422926_at | melanocortin 2 receptor | Mc2r # | 2.68 |
| 1424525_at | gastrin releasing peptide | Grp # | 2.54 |
| 1437466_at | activated leukocyte cell adhesion molecule | Alcam | 2.36 |
| 1449577_x_at | tropomyosin 2, beta | Tpm2 § | 2.35 |
| 1423100_at | FBJ osteosarcoma oncogene | Fos* | 2.15 |
| 1450468_at | Myocilin | Myoc | 2.13 |
| 1423062_at | insulin-like growth factor binding protein 3 | Igfbp3* | 2.05 |
| 1419693_at | collectin sub-family member 12 | Colec12 | 2.05 |
| 1433883_at | tropomyosin 4 | Tpm4 | 2.02 |
| 1452661_at | transferrin receptor | Tfrc | 1.99 |
| 1419871_at | Protein phosphatase 2 regulatory subunit A, beta isoform | Ppp2r1b b | 1.90 |
| 1449286_at | netrin G1 | Ntng1 | 1.87 |
| 1418445_at | solute carrier family 16, member 2 | Slc16a2 | 1.87 |
| 1425151_a_at | NADPH oxidase organizer 1 | Noxo1 | 1.85 |
| 1429262_at | Ras association (RaIGDS/AF-6) domain family member 6 | Rassf6 | 1.83 |
| 1437401_at | insulin-like growth factor 1 | Igf1 # | 1.79 |
| 1420647_a_at | keratin 8 | Krt8 | 1.78 |
| 1451912_a_at | fibroblast growth factor receptor-like 1 | Fgfrl1 | 1.77 |
| 1433486_at | chloride channel 3 | Clcn3 | 1.73 |
| 1427183_at | EGF-containing fibulin-like extracellular matrix protein 1 | Efemp1 | 1.71 |
| 1452890_at | tubulin tyrosine ligase-like family, member 5 | Ttll5 | 1.71 |
| 1433694_at | phosphodiesterase 3B, cGMP-inhibited | Pde3b | 1.71 |
| 1438610_a_at | crystallin, zeta | Cryz | 1.68 |
| 1447623_s_at | protein kinase D1 | Prkd1* | 1.68 |
| 1438292_x_at | adenosine kinase | Adk | 1.65 |
| 1435184_at | natriuretic peptide receptor 3 | Npr3 | 1.61 |
| 1452398_at | phospholipase C, epsilon 1 | Plce1 | 1.59 |
| 1451501_a_at | growth hormone receptor | Ghr # | 1.56 |
| 1448570_at | glia maturation factor, beta | Gmfb | 1.47 |
| 1422208_a_at | guanine nucleotide binding protein (G protein), beta 5 | Gnb5* | 1.45 |

**Table 5: Selected transcripts detected down-regulated in miR-143/145 KO vs. WT aortae. A selection of transcripts which was down-regulated in the Affymetrix GeneChip analysis is shown (p<0.05, n=5/5; see methods). Selected signaling molecules and markers of smooth muscle contraction are marked with # and *, respectively.**

| **Probe Set ID** | **Gene Title** | **Gene Symbol** | **FC** |
|---|---|---|---|
| 1417462_at | CAP, adenylate cyclase-associated protein 1 (yeast) | Cap1 | 0.14 |
| 1420942_s_at | regulator of G-protein signaling 5 | Rgs5* | 0.23 |
| 1421698_a_at | collagen, type XIX, alpha 1 | Col19a1 | 0.24 |
| 1449525_at | flavin containing monooxygenase 3 | Fmo3 | 0.27 |
| 1437559_at | regulator of G-protein signalling 7 binding protein | Rgs7bp* | 0.27 |
| 1431167_at | diacylglycerol kinase, gamma | Dgkg | 0.32 |
| 1446527_at | angiotensin II receptor, type 1 b | Agtr1b# | 0.34 |
| 1437933_at | Hedgehog-interacting protein protein phosphatase 1, regulatory (inhibitor) | Hhip | 0.35 |
| 1417701_at | subunit 14c phosphatidylinositol-4-phosphate 5-kinase, type 1 | Ppp1r14c | 0.37 |
| 1450389_s_at | beta | Pip5k1b | 0.39 |
| 1434887_at | frequenin homolog (Drosophila) | Freq | 0.41 |
| 1449522_at | unc-5 homolog C (C. elegans) | Unc5c | 0.41 |
| 1416286_at | regulator of G-protein signaling 4 potassium voltage-gated channel, Shal-related | Rgs4* | 0.42 |
| 1435720_at | family, member 3 | Kcnd3 | 0.42 |
| 1449876_at | protein kinase, cGMP-dependent, type I | Prkg1 | 0.45 |
| 1448598_at | matrix metallopeptidase 17 Rho-associated coiled-coil containing protein | Mmp17 | 0.45 |
| 1460729_at | kinase 1 | Rock1* | 0.45 |
| 1450700_at | CDC42 effector protein (Rho GTPase binding) 3 | Cdc42ep3* | 0.47 |
| 1423635_at | bone morphogenetic protein 2 | Bmp2# | 0.47 |
| 1455158_at | integrin alpha 3 betacellulin, epidermal growth factor family | Itga3 | 0.48 |
| 1435541_at | member | Btc | 0.49 |
| 1416357_a_at | melanoma cell adhesion molecule | Mcam | 0.50 |
| 1424768_at | caldesmon 1 | Cald1* | 0.50 |
| 1454159_a_at | insulin-like growth factor binding protein 2 CAP, adenylate cyclase-associated protein, 2 | Igfbp2* | 0.50 |
| 1423222_at | (yeast) | Cap2 | 0.52 |
| 1433489_s_at | fibroblast growth factor receptor 2 protein phosphatase 1, regulatory (inhibitor) | Fgfr2 | 0.52 |
| 1430286_s_at | subunit 14c | Ppp1r14c, CPI-17 | 0.54 |
| 1441042_at | fibroblast growth factor 1 | Fgf1 # | 0.54 |
| 1418413_at | caveolin 3 | Cav3 | 0.54 |
| 1423585_at | insulin-like growth factor binding protein 7 calcium channel, voltage-dependent, beta 2 | Igfbp7 | 0.56 |
| 1452476_at | subunit calcium/calmodulin-dependent protein kinase II | Cacnb2 | 0.57 |
| 1423942_a_at | gamma | Camk2g* | 0.58 |
| 1418744_s_at | similar to Tescalcin, tescalcin | Tesc | 0.58 |
| 1416701_at | Rho family GTPase 3 | Rnd3* | 0.58 |
| 1449660_s_at | coronin, actin binding protein 1C | Coro1c | 0.60 |
| 1423049_a_at | tropomyosin 1, alpha | Tpm1 | 0.61 |
| 1424051_at | collagen, type IV, alpha 2 | Col4a2 | 0.61 |
| 1422670_at | Rho family GTPase 2 | Rnd2* | 0.62 |
| 1433682_at | Rho guanine nucleotide exchange factor (GEF) 17 | Arhgef17* | 0.63 |
| 1423771_at | protein kinase C, delta binding protein | Prkcdbp | 0.64 |
| 1419161_a_at | NADPH oxidase 4 | Nox4 | 0.64 |
| 1424131_at | collagen, type VI, alpha 3 | Col6a3 | 0.64 |
| 1417312_at | dickkopf homolog 3 (Xenopus laevis) protein phosphatase 1, regulatory subunit 12A | Dkk3 | 0.65 |
| 1437734_at | (Ppp1r12a) | MYPT1* | 0.66 |
| 1425506_at | myosin, light polypeptide kinase | Mylk* | 0.70 |

**Table 6: Analysis of mRNA und protein expression in miR-143/145 knockout mice and identification of target proteins. (A) Transcriptional profiling of aortae from miR-143/145 knockout in comparison to WT mice (n=5/5) using Affymetrix GeneChip® Mouse Genome 430 2.0 arrays. Arrays were analyzed using the RMA algorithm. An unpaired t-test was performed for significance analysis. Fold changes and p-values are shown. (B) Quantitative comparative proteome analysis of protein extracts isolated from knockout and wild-type mouse aortae (n=3/3). Wild-type mice completely labeled with the 13C6Lys-substituted version of lysine ("SILAC mice") were used as a reference. Ratios of detected proteins were calculated using the maxquant 11.5 software. Ratios and p-values were calculated from the mean of three 13C6Lys-WT/WT and three 13C6Lys-WT/KO ratios. Lists of significantly up-regulated molecules were compared to predicted targets of mir-143 and miR-145 using the matchminer tool (18). Targets detected to be up-regulated by both unbiased screens at transcript- and protein level are in bold print; *, #, § label targets predicted by miRBase, miRNA.org or TargetScan, respectively.**

| **A) Selected target transcripts identified by Affymetrix GeneChip analysis** | | | | | | |
|---|---|---|---|---|---|---|
| Gene Title | | Symbol | p-value | FC | miRNA | database |
| Wnt inhibitory factor 1 | | Wif1 | 0.0095 | 3.8 | miR-145 | # |
| **activated leukocyte cell adhesion molecule** | | | | | | |
| | | **Alcam** | **0.0239** | **2.4** | **miR-145 miR-143, miR-145** | **#** |
| **tropomyosin 4** | | **Tpm4** | **0.0002** | **2.0** | miR-143, miR- | **#** |
| solute carrier family 16a2 | | Slc16a2 | 0.0015 | 1.9 | 145 | #§ |
| NADPH oxidase organizer 1 | | Noxo1 | 0.0162 | 1.8 | miR-145 | * |
| **crystallin, zeta** | | **Cryz** | **0.0041** | **1.7** | **miR-143** | **#*** |
| phospholipase C, epsilon 1 | | Plce1 | 0.0042 | 1.6 | miR-145 | *§ |
| **glia maturation factor, beta** | | **Gmfb** | **0.0002** | **1.5** | **miR-145** | **#§** |
| Rho GTPase activating protein 12 | | Arhgap12 | 0.0150 | 1.5 | miR-145 | # |
| **FK506 binding protein 3** | | **Fkbp3** | **0.0041** | **1.3** | **miR-145** | **#*§** |

| **B) Target proteins identified by SILAC analysis of protein expression** | | | | | | |
|---|---|---|---|---|---|---|
| Gene Title | | Symbol | p-value | ratio | miRNA | database |
| **crystallin, zeta** | | **Cryz** | **0.0271** | **9.2** | **miR-143** | **#^{*}** |
| N-myristoyltransferase | | Nmt1 | 0.0049 | 6.7 | miR-143 | § |
| **activated leukocyte cell adhesion molecule** | | | | | | |
| | | **Alcam** | **0.0351** | **5.5** | **miR-145** | **#** |
| Angiotensin-converting enzyme | | Ace | 0.0074 | 4.9 | miR-145 | * |
| myosin, heavy polypeptide 10, non-muscle | | Myh10 | 0.0108 | 3.6 | miR-143 | # |
| **FK506 binding protein 3** | | **Fkbp3** | **0.0430** | **3.4** | **miR-145** | **#*§** |
| Type I phosphodiesterase | | NPP1 | 0.0025 | 3.4 | miR-143, miR-145 | # |
| Versican core protein | | Vcan | 0.0291 | 2.7 | miR-143 | #^{*} |
| **glia maturation factor, beta** | | **Gmfb** | **0.0024** | **2.7** | **miR-145** | **#§** |
| Ubiquitin specific peptidase 5 | | Usp5 | 0.0153 | 2.4 | miR-145 | # |
| **Tropomyosin 4** | | **Tpm4** | **0.0466** | **2.1** | **miR-143, miR-145** | **#** |
| Platelet-activating factor acetylhydrolase beta IB | | | | | miR-143, miR-145 | |
| | | Pafah1b2 | 0.0252 | 2.0 | | # |
| Microtubule-associated protein 1B | Map1b | 0.0217 | 2.0 | miR-143 | #§ | |
| Platelet endothelial cell adhesion molecule | Pecam1 | 0.0392 | 1.7 | miR-145 | # | |

### REFERENCES

1. Owens, G.K., Kumar, M.S., and Wamhoff, B.R. 2004. Molecular regulation of vascular smooth muscle cell differentiation in development and disease. Physiol Rev 84:767-801.
2. Rensen, S.S., Doevendans, P.A., and van Eys, G.J. 2007. Regulation and characteristics of vascular smooth muscle cell phenotypic diversity. Neth Heart J 15:100-108.
3. Doran, A.C., Meller, N., and McNamara, C.A. 2008. Role of smooth muscle cells in the initiation and early progression of atherosclerosis. Arterioscler Thromb Vasc Biol 28:812-819.
4. Ross, R. 1995. Cell biology of atherosclerosis. Annu Rev Physiol 57:791-804.
5. Bartel, D.P. 2004. MicroRNAs: genomics, biogenesis, mechanism, and function. Cell 116:281-297.
6. Landgraf, P., Rusu, M., Sheridan, R., Sewer, A., lovino, N., Aravin, A., Pfeffer, S., Rice, A., Kamphorst, A.O., Landthaler, M., et al. 2007. A mammalian microRNA expression atlas based on small RNA library sequencing. Cell 129:1401-1414.
7. Zhao, Y., Ransom, J.F., Li, A., Vedantham, V., von Drehle, M., Muth, A.N., Tsuchihashi, T., McManus, M.T., Schwartz, R.J., and Srivastava, D. 2007. Dysregulation of cardiogenesis, cardiac conduction, and cell cycle in mice lacking miRNA-1-2. Cell 129:303-317.
8. Makeyev, E.V., Zhang, J., Carrasco, M.A., and Maniatis, T. 2007. The MicroRNA miR-124 promotes neuronal differentiation by triggering brain-specific alternative premRNA splicing. Mol Ce// 27:435-448.
9. Leucht, C., Stigloher, C., Wizenmann, A., Klafke, R., Folchert, A., and Bally-Cuif, L. 2008. MicroRNA-9 directs late organizer activity of the midbrain-hindbrain boundary. Nat Neurosci 11:641-648.
10. Ruzicka, D.L., and Schwartz, R.J. 1988. Sequential activation of alpha-actin genes during avian cardiogenesis: vascular smooth muscle alpha-actin gene transcripts mark the onset of cardiomyocyte differentiation. J Cell Biol 107:2575-2586.
11. Li, L., Miano, J.M., Cserjesi, P., and Olson, E.N. 1996. SM22 alpha, a marker of adult smooth muscle, is expressed in multiple myogenic lineages during embryogenesis. Circ Res 78:188-195.
12. Rishniw, M., Xin, H.B., Deng, K.Y., and Kotlikoff, M.I. 2003. Skeletal myogenesis in the mouse esophagus does not occur through transdifferentiation. Genesis 36:81-82.
13. Pfitzer, G., and Boels, P.J. 1991. Differential skinning of smooth muscle: a new approach to excitation-contraction coupling. Blood Vessels 28:262-267.
14. Khromov, A.S., Wang, H., Choudhury, N., McDuffie, M., Herring, B.P., Nakamoto, R., Owens, G.K., Somlyo, A.P., and Somlyo, A.V. 2006. Smooth muscle of telokindeficient mice exhibits increased sensitivity to Ca2+ and decreased cGMP induced relaxation. Proc Natl Acad Sci U S A 103:2440-2445.
15. Kruger, M., Moser, M., Ussar, S., Thievessen, I., Luber, C.A., Forner, F., Schmidt, S., Zanivan, S., Fassler, R., and Mann, M. 2008. SILAC mouse for quantitative proteomics uncovers kindlin-3 as an essential factor for red blood cell function. Cell 134:353-364.
16. John, B., Enright, A.J., Aravin, A., Tuschl, T., Sander, C., and Marks, D.S. 2004. Human MicroRNA targets. PLoS Biol 2:e363.
17. Betel, D., Wilson, M., Gabow, A., Marks, D.S., and Sander, C. 2008. The microRNA.org resource: targets and expression. Nucleic Acids Res 36:D149-153. 32
18. Griffiths-Jones, S., Saini, H.K., van Dongen, S., and Enright, A.J. 2008. miRBase: tools for microRNA genomics. Nucleic Acids Res 36:D154-158.
19. Grimson, A., Farh, K.K., Johnston, W.K., Garrett-Engele, P., Lim, L.P., and Bartel, D.P. 2007. MicroRNA targeting specificity in mammals: determinants beyond seed pairing. Mol Cell 27:91-105.
20. Bussey, K.J., Kane, D., Sunshine, M., Narasimhan, S., Nishizuka, S., Reinhold, W.C., Zeeberg, B., Ajay, W., and Weinstein, J.N. 2003. MatchMiner: a tool for batch navigation among gene and gene product identifiers. Genome Biol 4:R27.
21. Abouhamed, M., Reichenberg, S., Robenek, H., and Plenz, G. 2003. Tropomyosin 4 expression is enhanced in dedifferentiating smooth muscle cells in vitro and during atherogenesis. Eur J Cell Biol 82:473-482.
22. Potapova, I.A., Cohen, I.S., and Doronin, S.V. 2007. Voltage-gated ion channel Kv4.3 is associated with Rap guanine nucleotide exchange factors and regulates angiotensin receptor type 1 signaling to small G-protein Rap. FEBS J 274:4375-4384.
23. Miralles, F., Posern, G., Zaromytidou, A.I., and Treisman, R. 2003. Actin dynamics control SRF activity by regulation of its coactivator MAL. Cell 113:329-342.
24. Ji, R., Cheng, Y., Yue, J., Yang, J., Liu, X., Chen, H., Dean, D.B., and Zhang, C. 2007. MicroRNA expression signature and antisense-mediated depletion reveal an essential role of MicroRNA in vascular neointimal lesion formation. Circ Res 100:1579-1588.
25. Xu, Q. 2004. Mouse models of arteriosclerosis: from arterial injuries to vascular grafts. Am J Pathol 165:1-10.
26. Farh, K.K., Grimson, A., Jan, C., Lewis, B.P., Johnston, W.K., Lim, L.P., Burge, C.B., and Bartel, D.P. 2005. The widespread impact of mammalian MicroRNAs on mRNA repression and evolution. Science 310:1817-1821.
27. Wang, D., Chang, P.S., Wang, Z., Sutherland, L., Richardson, J.A., Small, E., Krieg, P.A., and Olson, E.N. 2001. Activation of cardiac gene expression by myocardin, a transcriptional cofactor for serum response factor. Cell 105:851-862.
28. Xu, N., Papagiannakopoulos, T., Pan, G., Thomson, J.A., and Kosik, K.S. 2009. MicroRNA-145 regulates OCT4, SOX2, and KLF4 and represses pluripotency in human embryonic stem cells. Cell 137:647-658.
29. Dzau, V.J. 2001. Theodore Cooper Lecture: Tissue angiotensin and pathobiology of vascular disease: a unifying hypothesis. Hypertension 37:1047-1052.
30. Touyz, R.M. 2005. Intracellular mechanisms involved in vascular remodelling of resistance arteries in hypertension: role of angiotensin II. Exp Physiol 90:449-455.
31. Schieffer, B., Schieffer, E., Hilfiker-Kleiner, D., Hilfiker, A., Kovanen, P.T., Kaartinen, M., Nussberger, J., Harringer, W., and Drexler, H. 2000. Expression of angiotensin II and interleukin 6 in human coronary atherosclerotic plaques: potential implications for inflammation and plaque instability. Circulation 101:1372-1378.
32. Griendling, K.K., Delafontaine, P., Rittenhouse, S.E., Gimbrone, M.A., Jr., and Alexander, R.W. 1987. Correlation of receptor sequestration with sustained diacylglycerol accumulation in angiotensin II-stimulated cultured vascular smooth muscle cells. J Biol Chem 262:14555-14562.
33. Lassegue, B., Alexander, R.W., Nickenig, G., Clark, M., Murphy, T.J., and Griendling, K.K. 1995. Angiotensin II down-regulates the vascular smooth muscle AT1 receptor by transcriptional and posttranscriptional mechanisms: evidence for homologous and heterologous regulation. Mol Pharmacol 48:601-609.
34. Wang, X., Adams, L.D., Pabon, L.M., Beaudry, D., Gunaje, J., Geary, R.L., Deblois, D., and Schwartz, S.M. 2007. RGS5, RGS4, and RGS2 expression and aortic contractibility are dynamically co-regulated during aortic banding-induced hypertrophy. J Mol Cell Cardiol. 33
35. Hercule, H.C., Tank, J., Plehm, R., Wellner, M., da Costa Goncalves, A.C., Gollasch, M., Diedrich, A., Jordan, J., Luft, F.C., and Gross, V. 2007. Regulator of G protein signalling 2 ameliorates angiotensin II-induced hypertension in mice. Exp Physiol 92:1014-1022.
36. Lyssand, J.S., DeFino, M.C., Tang, X.B., Hertz, A.L., Feller, D.B., Wacker, J.L., Adams, M.E., and Hague, C. 2008. Blood pressure is regulated by an alpha1Dadrenergic receptor/dystrophin signalosome. J Biol Chem 283:18792-18800.
37. Krege, J.H., Kim, H.S., Moyer, J.S., Jennette, J.C., Peng, L., Hiller, S.K., and Smithies, O. 1997. Angiotensin-converting enzyme gene mutations, blood pressures, and cardiovascular homeostasis. Hypertension 29:150-157.
38. Seitz, H. 2009. Redefining microRNA targets. Curr Biol 19:870-873.
39. Penn, B.H., Bergstrom, D.A., Dilworth, F.J., Bengal, E., and Tapscott, S.J. 2004. A MyoD-generated feed-forward circuit temporally patterns gene expression during skeletal muscle differentiation. Genes Dev 18:2348-2353.
40. Stary, H.C., Chandler, A.B., Glagov, S., Guyton, J.R., Insull, W., Jr., Rosenfeld, M.E., Schaffer, S.A., Schwartz, C.J., Wagner, W.D., and Wissler, R.W. 1994. A definition of initial, fatty streak, and intermediate lesions of atherosclerosis. A report from the Committee on Vascular Lesions of the Council on Arteriosclerosis, American Heart Association. Circulation 89:2462-2478.
41. Ip, J.H., Fuster, V., Badimon, L., Badimon, J., Taubman, M.B., and Chesebro, J.H. 1990. Syndromes of accelerated atherosclerosis: role of vascular injury and smooth muscle cell proliferation. J Am Coll Cardiol 15:1667-1687.
42. Ross, R., and Glomset, J.A. 1973. Atherosclerosis and the arterial smooth muscle cell: Proliferation of smooth muscle is a key event in the genesis of the lesions of atherosclerosis. Science 180:1332-1339.
43. Daugherty, A., Manning, M.W., and Cassis, L.A. 2000. Angiotensin II promotes atherosclerotic lesions and aneurysms in apolipoprotein E-deficient mice. J Clin Invest 105:1605-1612.
44. Halkin, A., and Keren, G. 2002. Potential indications for angiotensin-converting enzyme inhibitors in atherosclerotic vascular disease. Am J Med 112:126-134.
45. Warming, S., Costantino, N., Court, D.L., Jenkins, N.A., and Copeland, N.G. 2005. Simple and highly efficient BAC recombineering using galK selection. Nucleic Acids Res 33:e36.
46. Voss, A.K., Thomas, T., and Gruss, P. 1997. Germ line chimeras from female ES cells. Exp Cell Res 230:45-49.
47. Scholz, D., Ito, W., Fleming, I., Deindl, E., Sauer, A., Wiesnet, M., Busse, R., Schaper, J., and Schaper, W. 2000. Ultrastructure and molecular histology of rabbit hind-limb collateral artery growth (arteriogenesis). Virchows Arch 436:257-270.
48. Cox, J., and Mann, M. 2007. Is proteomics the new genomics? Cell 130:395-398.
49. Graumann, J., Hubner, N.C., Kim, J.B., Ko, K., Moser, M., Kumar, C., Cox, J., Scholer, H., and Mann, M. 2008. Stable isotope labeling by amino acids in cell culture (SILAC) and proteome quantitation of mouse embryonic stem cells to a depth of 5,111 proteins. Mol Cell Proteomics 7:672-683.
50. Kersey, P.J., Duarte, J., Williams, A., Karavidopoulou, Y., Birney, E., and Apweiler, R. 2004. The International Protein Index: an integrated database for proteomics experiments. Proteomics 4:1985-1988.
51. Brede, M., Wiesmann, F., Jahns, R., Hadamek, K., Arnolt, C., Neubauer, S., Lohse, M.J., and Hein, L. 2002. Feedback inhibition of catecholamine release by two different alpha2-adrenoceptor subtypes prevents progression of heart failure. Circulation 106:2491-2496.
52. Brede, M., Hadamek, K., Meinel, L., Wiesmann, F., Peters, J., Engelhardt, S., Simm, A., Haase, A., Lohse, M.J., and Hein, L. 2001. Vascular hypertrophy and increased P70S6 kinase in mice lacking the angiotensin II AT(2) receptor. Circulation 104:2602-2607.
53. Fabiato, A., and Fabiato, F. 1979. Calculator programs for computing the composition of the solutions containing multiple metals and ligands used for experiments in skinned muscle cells. J Physiol (Paris) 75:463-505.

## Claims

1. A nucleic acid molecule for use in the treatment or preventive treatment of a patient after stent implantation or balloon dilatation, wherein the nucleic acid molecule is selected from
(a) a single-stranded nucleic acid molecule comprising or consisting of the sequence of SEQ ID NO: 1, 2, 3 or 4 or a sequence having at least 90% sequence identity to the sequence of SEQ ID NO: 1, 2, 3 or 4;
(b) a hairpin RNA, wherein one of the regions forming the double-stranded portion of said hairpin RNA comprises or consists of the sequence of SEQ ID NO: 1, 2, 3 or 4 or a sequence having at least 90% sequence identity to the sequence of SEQ ID NO: 1, 2, 3 or 4;
(c) an at least partially double-stranded RNA comprising two separate single strands, wherein a region within one of the strands, said region being located within the double-stranded portion of said double-stranded RNA, comprises or consists of the sequence of SEQ ID NO: 1, 2, 3 or 4 or a sequence having at least 90% sequence identity to the sequence of SEQ ID NO: 1, 2, 3 or 4;
(d) a nucleic acid molecule encoding the nucleic acid molecule of (a) or the RNA (b); and
(e) a nucleic acid molecule or a two nucleic acid molecules encoding the two separate single strands of the RNA of (c).

2. The nucleic acid molecule as defined in claim 1 for use in the preventive treatment of a patient after stent implantation or balloon dilatation, wherein the treatment is for the prevention of restenosis.

3. The nucleic acid molecule as defined in claim 1 for use in the treatment or preventive treatment of a patient after stent implantation or balloon dilatation, wherein said treatment or preventive treatment is for the prevention or treatment of thrombosis.

4. The nucleic acid molecule as defined in claim 1 for use in the treatment or preventive treatment of a patient after stent implantation or balloon dilatation, wherein said treatment or preventive treatment is for the prevention or treatment of hypertension.

5. The nucleic acid molecule as defined in claim 1 or 2, wherein the nucleic acid molecule is fused to a lipid.

6. The nucleic acid molecule as defined in claim 3, wherein the lipid is a cholesterol.

7. A drug eluting stent comprising a nucleic acid molecule as defined in any of claims 1 or 6.

8. An in vitro method of identifying an activator of expression of a microRNA, said microRNA comprising or consisting of the sequence of SEQ ID NO: 1, 2, 3 or 4 or a sequence having at least 90% sequence identity to the sequence of SEQ ID NO: 1, 2, 3 or 4, said method comprising:
(a) providing a cell or an in vitro expression system, said cell or system being capable of expressing said microRNA under the control of the native promoter;
(b) bringing into contact said cell or system with a test compound; and
(c) comparing the expression level of said microRNA after and prior to step (b), wherein an increase of the expression level is indicative of said compound being an activator of expression of said microRNA,
wherein said activator is a pharmaceutically active agent for the treatment or preventive treatment of a patient after stent implantation or balloon dilatation, or a lead compound suitable for developing a pharmaceutically active agent for the treatment or preventive treatment of a patient after stent implantation or balloon dilatation.
